# EUROPEAN PATENT APPLICATION

(11) **EP 4 375 299 A1**
(43) Date of publication of application: **29.05.2024**
(21) Application number: 22845319.7
(22) Date of filing: 19.07.2022
(51) Int. Cl.: C07K 16/28, C07K 16/46, A61K 39/395, A61P 35/00

(54) **B7-H3 ANTIBODY AND USE THEREOF**

(30) Priority: 20.07.2021 CN 202110817833
(71) Applicant: EXCELMAB INC., Guangzhou, Guangdong 510000 (CN)
(72) Inventor: ZHANG, Wenjun, Guangzhou, Guangdong 510000 (CN); LIU, Min, Guangzhou, Guangdong 510000 (CN); TIAN, Wenwu, Guangzhou, Guangdong 510000 (CN); LI, Feng, Guangzhou, Guangdong 510000 (CN); HUA, Yanan, Guangzhou, Guangdong 510000 (CN)
(74) Representative: Rommeswinkel, Marike
(86) International application number: PCT/CN2022/106546
(87) International publication number: WO 2023/001154

(57) **Abstract**

The present application provides a B7-H3 antibody and a use thereof The heavy chain complementarity determining regions of the B7-H3 antibody comprise the amino acid sequences shown in SEQ ID NOs. 6-8, and the light chain complementarity determining regions of the B7-H3 antibody comprise the amino acid sequences shown in SEQ ID NOs. 14-16. The B7-H3 antibody can bind, with high affinity, to a B7-H3 antigen and cells expressing the B7-H3 antigen, and can efficiently mediate PBMC to kill B7-H3 positive tumors. A B7-H3×CD3 bispecific antibody is constructed by using the B7-H3 antibody. The bispecific antibody exhibits high affinity and stability, and can effectively mediate PBMC to kill tumor cells.

## Description

### Cross-Reference to Related Applications

This invention claims priority to the Chinese patent application with the filing No. CN202110817833.7 filed to China National Intellectual Property Administration on July 20, 2021, which is entitled "B7-H3 Antibody and Use Thereof', the contents of which are incorporated herein by reference in entirety.

### Technical Field

This invention belongs to the technical field of tumor treatment, and molecular immunology, and relates to a B7-H3 antibody and use thereof.

### Technical Background

B7-H3 (B7 homolog 3 protein, also known as CD276), a member of the B7/CD28 immunoglobulin superfamily, is a single-pass transmembrane protein (Steinberger P, Majdic O, Derdak S V, et al. Molecular Characterization of Human 4Ig-B7-H3, a Member of the B7 Family with Four Ig-Like Domains Journal of Immunology, 2004, 172(4):2352-2359.). B7-H3 consists of 316 amino acids, which includes a signal peptide at an amino terminus, extracellular immunoglobulin-like variable regions (IgV), constant regions (IgC), a transmembrane region, and a cytoplasmic region of 45 amino acids, and it is expressed in monocytes, dendritic cells, and activated T cells.

Currently, the receptor for B7-H3 has not been confirmed. Some studies suggest that B7-H3 receptor is a type I transmembrane protein TLT-2, which belongs to the triggering receptor expressed on myeloid cells (TREM) family (King R G, Herrin B R, Justement L B. Trem-like transcript 2 is expressed on cells of the myeloid/granuloid and B lymphoid lineage and is up-regulated in response to inflammation. Journal of Immunology, 2006, 176(10):6012-6021). The functions of TREM family molecules are to regulate cellular response, and exert an effect on innate and acquired immunity (Klesney-Tait J, Turnbull I R, Colonna M. The TREM receptor family and signal integration. Nature Immunology, 2006, 7(12):1266). TLT-2 protein is expressed on CD8+ T-cells constitutively and also on activated CD4+ T cells.

B7-H3 possesses dual actions as both a co-stimulatory/co-inhibitory molecule. When anti-CD3 antibodies are present, B7-H3 can promote the proliferation of CD4+ T cell and CD8+ T cell populations, and selectively stimulates the production of γ-interferon (IFN-γ). When TLT-2 is transfected into T cells, it can promote the production of interleukin (IL)-2 and IFN-γ through interaction with B7-H3. Blocking the interaction between B7-H3 and TLT-2 can inhibit hypersensitivity mediated by the CD8+ T cells. On the other hand, experiments in mice have suggested that B7-H3 also has a co-inhibitory function, and results in tumor escape from immune response. B7-H3 can also inhibit NK cell activity *in vitro,* thereby reducing the function of NK cells.

B7-H3 is expressed in a low level on normal tissues but is over-expressed in a variety of cancers, especially in nonsmall-cell lung cancer, kidney cancer, urothelial cancer, colorectal cancer, prostate cancer, glioblastoma multiforme, ovarian cancer, and pancreatic cancer. B7-H3 plays an important role in tumor immune escape through the inhibitory effect on the T cells and has been proved of promoting tumor progression and cancer cell metastasis. In addition, B7-H3 is expressed not only on cancer cells, but also on tumor-associated vasculature. B7-H3 is also involved in the pathogenesis of auto-immune diseases. In rheumatic arthritis and other autoimmune diseases, B7-H3 plays an important role in the interactions between fibroblast-like synoviocytes and activated T cells (Tran C N, Thacker S G, Louie D M, et al. Interactions of T Cells with Fibroblast-Like Synoviocytes: Role of the B7 Family Costimulatory Ligand B7-H3. Journal of Immunology, 2008, 180(5):2989-2998.). Moreover, B7-H3 acts as a costimulatory factor when macrophages release cytokines, and thus is related to emergence of sepsis.

At present, there is a demand to develop new B7-H3 antibodies as a therapeutic agent in the fields of immunotherapy and cancer treatment.

### Summary

The present invention discloses a B7-H3 antibody, wherein heavy chain complementarity determining regions of the B7-H3 antibody include amino acid sequences as set forth in SEQ ID Nos. 6-8; and
light chain complementarity determining regions of the B7-H3 antibody contain amino acid sequences as set forth in SEQ ID Nos. 14-16.

In some embodiments, heavy chain variable regions of the B7-H3 antibody contain an amino acid sequence as set forth in SEQ ID No. 1; and
light chain variable regions of the B7-H3 antibody contain an amino acid sequence as set forth in SEQ ID No. 9.

This invention further provides an affinity-matured B7-H3 antibody, wherein the affinity-matured B7-H3 antibody is prepared from the B7-H3 antibody through amino acid mutation.

In some embodiments, a heavy chain complementarity determining region CDR1 of the affinity-matured B7-H3 antibody includes an amino acid sequence as set forth in SEQ ID No. 6 or one of SEQ ID Nos. 17-25, a heavy chain complementarity determining region CDR2 includes an amino acid sequence as set forth in SEQ ID No. 7 or one of SEQ ID Nos. 26-37, and a heavy chain complementarity determining region CDR3 includes an amino acid sequence as set forth in SEQ ID No. 8 or one of SEQ ID Nos. 38-48; and
light chain complementarity determining regions of the affinity-matured B7-H3 antibody include amino acid sequences as set forth in SEQ ID Nos. 14-16.

In some embodiments, light chain variable regions of the affinity-matured B7-H3 antibody include an amino acid sequence as set forth in SEQ ID No. 9; and heavy chain framework regions of the affinity-matured B7-H3 antibody include amino acid sequences as set forth in SEQ ID Nos. 2-5.

This invention further provides a bispecific antibody, wherein the bispecific antibody includes two heavy chains and two light chains;

The two heavy chains include an anti-B7-H3 heavy chain and an anti-CD3 heavy chain; the anti-B7-H3 heavy chain includes a heavy chain of the B7-H3 antibody or a heavy chain of the affinity-matured B7-H3 antibody in any one of the above;

The two light chains include an anti-B7-H3 light chain and an anti-CD3 light chain; the anti-B7-H3 light chain includes a light chain of the B7-H3 antibody or a light chain of the affinity-matured B7-H3 antibody in any one of the above;

The heavy chains bind to the light chains through disulfide bonds; and
the anti-B7-H3 heavy chain binds to the anti-CD3 heavy chain through disulfide bonds.

In some embodiments, a variable region of the anti-CD3 heavy chain includes an amino acid sequence as set forth in SEQ ID No. 49; and
a variable region of the anti-CD3 light chain includes an amino acid sequence as set forth in SEQ ID No. 9.

In some embodiments, the bispecific antibody includes an immunoglobulin Fab domain binding to the B7-H3, an immunoglobulin Fab domain binding to the CD3, and a heterodimeric Fc region;

The immunoglobulin Fab domain binding to B7-H3 includes a variable region VH and a constant region CH1 of the anti-B7-H3 heavy chain as well as a variable region and constant region of the anti-B7-H3 light chain;

The immunoglobulin Fab domain binding to CD3 includes a variable region VH and a constant region CH1 of the anti-CD3 heavy chain as well as a variable region and constant region of the anti-CD3 light chain; and

The heterodimeric Fc region includes an Fc fragment linked to the anti-B7-H3 heavy chain and an Fc fragment linked to the anti-CD3 heavy chain.

In some embodiments, the Fc fragment linked to the anti-B7-H3 heavy chain includes a human Fc fragment or a humanized Fc fragment.

In some embodiments, CH3 of the Fc fragment linked to the anti-B7-H3 heavy chain contains T394D, P395D, and P396D mutations.

In some embodiments, CH3 of the Fc fragment linked to the anti-CD3 heavy chain contains P395K, P396K, and V397K mutations.

In some embodiments, CH2 of the Fc fragment linked to the anti-B7-H3 heavy chain contains L234A, L235A, and P329G mutations.

This invention further provides a biomaterial, wherein the biomaterial is any one of the following B1) to B6):
B1) a nucleic acid molecule encoding the antibody of any one of (b1) to (b3), or the heavy chain and/or light chain in the antigen-binding domain of the antibody of any one of (b1) to (b3): (b1) is the B7-H3 antibody in the above, (b2) is any one of the affinity-matured B7-H3 antibodies described above, or (b3) is any one of the bispecific antibodies described above;
B2) an expression cassette containing the nucleic acid molecule described in B1);
B3) a recombinant vector containing the nucleic acid molecule described in B1), or a recombinant vector containing the expression cassette described in B2);
B4) a recombinant microorganism containing the nucleic acid molecule described in B1), or a recombinant microorganism containing the expression cassette described in B2), or a recombinant microorganism containing the recombinant vector described in B3);
B5) a cell line containing the nucleic acid molecule described in B1), or a cell line containing the expression cassette described in B2), or a cell line containing the recombinant vector described in B3); and
B6) a nucleic acid molecule encoding the antibody of any one of (b1) to (b3), or the variable region of heavy chain and/or the variable region of light chain in the antigen-binding domain of the antibody of any one of (b1) to (b3).

This invention further provides a pharmaceutical composition, wherein the pharmaceutical composition includes any one or a combination of at least two of the B7-H3 antibody, the affinity-matured B7-H3 antibody in any one of the above, or the bispecific antibody in the above.

This invention further provides use of the B7-H3 antibody, the affinity-matured B7-H3 antibody in any one of the above, the bispecific antibody in any one of the above, or the pharmaceutical composition in preparation of an anti-tumor drug; and

The tumor types include any one of neurological system tumor, colorectal cancer, liver cancer, head and neck cancer, lung cancer, melanoma, pancreatic cancer, gastric cancer, kidney cancer, bladder cancer, breast cancer, ovarian cancer or prostate cancer.

This invention further provides use of the B7-H3 antibody, the affinity-matured B7-H3 antibody in any one of the above, the bispecific antibody in any one of the above, or the pharmaceutical composition in tumor treatment.

In some embodiments, the tumor includes any one of neurological system tumor, colorectal cancer, liver cancer, head and neck cancer, lung cancer, melanoma, pancreatic cancer, gastric cancer, kidney cancer, bladder cancer, breast cancer, ovarian cancer or prostate cancer.

This invention further provides a method for treating a tumor-associated disease, wherein the B7-H3 antibody, the affinity-matured B7-H3 antibody in any one of the above, the bispecific antibody in any one of the above, or the pharmaceutical composition is administered to a subject in need thereof.

In some embodiments, the tumor includes any one of neurological system tumor, colorectal cancer, liver cancer, head and neck cancer, lung cancer, melanoma, pancreatic cancer, gastric cancer, kidney cancer, bladder cancer, breast cancer, ovarian cancer or prostate cancer.

### Brief Description of Drawings

FIG. 1 is a graph of binding activities of 5D9 to B7-H3 antigen, analyzed by ELISA;
FIG. 2A is a graph of binding activities of affinity-matured variants (44F2, 43G11, 46H7, and 45B11) to B7-H3 antigen, analyzed by ELISA;
FIG. 2B is a graph of binding activities of affinity-matured variants (43D4, 42F7, and 45F4) to B7-H3 antigen, analyzed by ELISA;
FIG. 2C is a graph of binding activities of affinity-matured variants (46H9, 47H4, 46D4, and 43G5) to B7-H3 antigen, analyzed by ELISA;
FIG. 2D is a graph of binding activities of affinity-matured variants (44C5, 42A1, and 47C8) to B7-H3 antigen, analyzed by ELISA;
FIG. 2E is a graph of binding activities of affinity-matured variants (48E9, 49C3, 49C8, and 49E1) to B7-H3 antigen, analyzed by ELISA;
FIG. 2F is a graph of binding activities of affinity-matured variants (49F6, 50A1, 50E3, and 49H5) to B7-H3 antigen, analyzed by ELISA;
FIG. 2G is a graph of binding activities of affinity-matured variants (50B10, 48G2, 49D12, and 48D10) to B7-H3 antigen, analyzed by ELISA;
FIG. 2H is a graph of binding activities of affinity-matured variants (48A6, 48B12, 49B2, and 49C7) to B7-H3 antigen, analyzed by ELISA;
FIG. 2I is a graph of binding activities of affinity-matured variants (50A4, 50A6, and 50A11) to B7-H3 antigen, analyzed by ELISA;
FIG. 3A is a graph of binding activities of affinity-matured variants (44F2 and 43G11) to cells overexpressing B7-H3 membrane-proximal domain, analyzed by FACS;
FIG. 3B is a graph of binding activities of affinity-matured variants (45B11, 43D4, and 46H7) to cells overexpressing B7-H3 membrane-proximal domain, analyzed by FACS;
FIG. 3C is a graph of binding activities of affinity-matured variants (43G5, 42F7, and 45F4) to cells overexpressing B7-H3 membrane-proximal domain, analyzed by FACS;
FIG. 3D is a graph of binding activities of affinity-matured variants (46H9, 47H4, and 46D4) to cells overexpressing B7-H3 membrane-proximal domain, analyzed by FACS;
FIG. 3E is a graph of binding activities of affinity-matured variants (44C5, 42A1, and 47C8) to cells overexpressing B7-H3 membrane-proximal domain, analyzed by FACS;
FIG. 4A is a graph of killing activity of PBMC on B7-H3 positive tumors, mediated by affinity-matured antibody variants (44F2, 43G11, 46H7, 45B11, 43D4, 42F7, 45F4, and 46H9);
FIG. 4B is a graph of killing activity of PBMC on B7-H3 positive tumors, mediated by affinity-matured antibody variants (46D4, 47H4, 48E9, 49C3, 49E1, 50A1, 50A6, and 5D9);
FIG. 5 is a structural diagram of B7-H3×CD3 bispecific antibody molecule;
FIG. 6 is a graph of binding activity of B7-H3×CD3 bispecific antibodies;
FIG. 7 is a graph of T cell activation induced by B7-H3×CD3 bispecific antibodies;
FIG. 8A is a graph of activity of B7-H3×CD3 bispecific antibodies (49E1×CD3, 42F7×CD3, 43G11×CD3, 8H9×CD3, and 46H7×CD3) mediated PBMC to kill NCI-N87 cells;
FIG. 8B is a graph of activity of B7-H3×CD3 bispecific antibodies (42F7×CD3, 43G11×CD3, 49E1×CD3, 8H9×CD3, and 46H7×CD3) mediated PBMC to kill A498 cells;
FIG. 8C is a graph of B7-H3×CD3 bispecific antibodies (42F7×CD3, 43G11×CD3, 49E1×CD3, 8H9×CD3, and 46H7×CD3) mediated PBMC to kill HepG2 cells;
FIG. 9A is a graph of the stability study result of 46H7×CD3 bispecific antibody;
FIG. 9B is a graph of the stability study result of 43G11×CD3 bispecific antibody;
FIG. 9C is a graph of the stability study result of 49E1×CD3 bispecific antibody;
FIG. 9D is a graph of the stability study result of 42F7×CD3 bispecific antibody; and
FIG. 10 is a graph of anti-tumor activity of B7-H3×CD3 bispecific antibody in xenograft mice model.

### Detailed Description of Embodiments

In order to further illustrate technical solutions adopted in this invention and effects thereof, this invention is further described as below in combination with embodiments, examples, and drawings. It is understood that the embodiments described herein are merely used to explain this invention rather than to limit the scope of this invention.

Where specific techniques or conditions are not specified in the embodiments or examples, they are carried out according to techniques or conditions described in documents in the art or according to product specifications. Where manufacturers of reagents or apparatuses used are not specified, they are all conventional products commercially available through normal channels.

Unless otherwise defined, the scientific and technical terms and their abbreviations thereof used in this invention should have meanings that are commonly understood by those ordinarily skilled in the art. Part of the terms and abbreviations used in this invention are listed below.

Antibody, Ab.

Immunoglobulin, Ig.

Heavy chain, HC.

Light chain, LC.

Heavy chain variable domain (Heavy chain variable region), VH.

Heavy chain constant domain (Heavy chain constant region), CH.

Light chain variable domain (Light chain variable region), VL.

Light chain constant domain (Light chain constant region), CL.

Antigen binding fragment, Fab.

Hinge region.

Fc Fragment: Fragment crystallizable region, Fc region.

Monoclonal antibodies, mAbs.

Antibody-dependent cell-mediated cytotoxicity, ADCC.

Complement dependent cytotoxicity, CDC.

Natural killing cell, NK cell.

Bispecific antibody, BsAb.

T cell receptor, TCR.

Major histocompatibility complex, MHC.

Complementarity determining region, CDR, referring to antigen complementarity binding region of antibody.

Immunoreceptor tyrosine-based activation motif, ITAM.

Single-chain variable antibody fragment (also called as single-chain antibody): single-chain variable fragment, scFv;
Adoptive cellular immunotherapy, ACI.

Lymphokine-activated killer cell, LAK cell.

Tumor Infiltrating Lymphocyte, TIL cell.

Cytokine-induced killer cell, CIK cell.

Protocol steps of experiments such as molecular cloning, cell culture, protein purification, immunological experiments, microbiology, and animal models described in this invention are routine steps widely used in the art. Singular terms in this invention include pluralities and plural meanings include singularity meanings, unless otherwise stated in context. Nucleotide sequences described in this invention are arranged and written from left to right in a direction from a 5' end to a 3' end, unless otherwise specified. Amino acid sequences in this invention are arranged and written from left to right in a direction from an amino terminus (N-terminus) to a carboxyl terminus (C-terminus), unless otherwise specified. Three-letter abbreviations for amino acids and one-letter abbreviations for nucleotides mentioned in this invention are in the forms generally accepted in the technical field, and one-letter abbreviations for amino acids are in the forms recommended by the IUPAC-IUB Biochemical Nomenclature Commission.

The term "amino acid" refers to one of the 20 naturally occurring amino acids or any non-natural analogs that may be present at a specific defined position. The term "amino acid mutation" described in this invention refers to an amino acid substitution, insertion, deletion, or modification in a polypeptide sequence, or any combination of the amino acid substitution, insertion, deletion, and modification. A preferred amino acid modification herein is a substitution. In this invention, "amino acid substitution" or "substitution" refers to replacement of an amino acid at a particular position in a parent polypeptide sequence with another amino acid. For example, substitution C220S refers to a variant polypeptide, in which amino acid Cysteine at position 220 of the polypeptide has been replaced with amino acid Serine. Amino acid mutations may be achieved by molecular cloning or chemical methods. The molecular cloning methods include PCR, site-directed mutagenesis, full gene synthesis, etc.

The terms "protein", "peptide chain", and "polypeptide chain" refer to molecules in which two or more amino acids are linked by peptide bonds, including natural proteins, artificial proteins, protein fragments, mutant proteins, fusion proteins, and the like.

The term "domain" refers to a specific structural region with independent functions in a biological macromolecule. A domain has an independent tertiary structure and its function does not depend on the remaining moiety of the biological macromolecule. The domain in this invention refers particularly to a region in a protein, such as a domain of a heavy chain variable region VH or a domain of a light chain variable region VL. Domains may bind to each other to form a large domain.

The term "antibody" refers to an immunoglobulin molecule containing at least one antigen-recognition site and capable of specifically binding to an antigen. Herein, the term "antigen" refers to a substance that can induce an immune response in a body and specifically binds to an antibody, such as protein, polypeptide, peptide, carbohydrate, polynucleotide, lipid, or hapten, or a combination thereof. The binding of an antibody to an antigen is mediated by the interaction formed therebetween, including a hydrogen bond, a van der Waals force, an ionic bond, and a hydrophobic bond. The region on the surface of an antigen that binds to an antibody is called an "antigenic determinant" or "epitope". Generally, each antigen has multiple determinants. The term "antibody" mentioned in this invention includes monoclonal antibody (including full-length monoclonal antibody), polyclonal antibody, antibody fragment, multispecific antibody (e.g., bispecific antibody) containing at least two different epitope binding domains, human antibody, humanized antibody, posttranslationally modified antibody, camelid antibody, chimeric antibody, fusion protein containing antibody antigenic determinants, and any other modified immunoglobulin molecule containing antigen recognition sites, as long as these antibodies exhibit desired biological activity. Antibodies include immunoglobulin molecules and immunologically active fragments of immunoglobulin molecules, namely molecules containing at least one antigen-binding site.

The term "bispecific antibody" refers to an antibody containing two different antigen-binding sites, and can simultaneously bind to two different antigen-binding sites.

The term "Fab", "Fab region", "Fab fragment", or "Fab molecule" refers to an antigen-binding fragment, containing a VH domain and a CH1 domain of a heavy chain and a VL domain and a CL domain of a light chain of an immunoglobulin, where a first constant region domain CH1 of the heavy chain binds to the constant region domain CL of the light chain, and the variable region domain VH of the heavy chain binds to the variable region domain VL of the light chain.

The term "Fc", "Fc region", "Fc fragment", or "Fc molecule" refers to an effector region of an antibody, which can induce, for example, CDC, ADCC, ADCP, and cytokine release. The Fc of a natural antibody is usually formed by binding two identical protein fragments, each containing two or three immunoglobulin constant region domains. The Fc described in this invention includes a natural Fc and a mutated Fc. Although boundaries of the Fc region may vary, the Fc region of a human IgG heavy chain is usually defined to include residues from C226 or P230 to its carboxyl terminus. Under experimental conditions, fragments generated by enzymatic cleavage of an immunoglobulin monomer by papain are Fab and Fc, respectively. The "hinge" or "hinge region" of an antibody refers to a flexible polypeptide containing amino acids between the first and second constant domains (CH1 and CH2) of the antibody.

Amino acids of a variable region of an antibody described in the present disclosure are numbered by using the encoding scheme set forth by Kabat et al. in 1991, namely, the "Kabat index" or "Kabat numbering" (Kabat, E. A. et al. Sequences of Proteins of Immunological Interest, 5th ed., NIH Publication No. 91-3242, Bethesda, MD.:1991), unless otherwise specified. Amino acids of a constant region of an antibody described in the present disclosure are numbered by using the EU index (Edelman GM, et.al. Proc Natl Acad Sci U S A 1969,63:78-85.), unless otherwise specified.

The term "antigen-binding site" refers to one or more amino acid residues of an antigen-binding molecule that directly interact with an antigen. The antigen-binding site of an antibody is composed of an antigen complementarity determining region (CDR). An innate immunoglobulin molecule usually contains two antigen-binding sites. A Fab molecule usually contains one antigen-binding site.

The term "T cell activation (or activation of T cells)" refers to one or more immune responses of T lymphocytes, especially killer T lymphocytes, including: proliferation, differentiation, release of cytokines, secretion of killer effector molecules, cell killing, etc.

The term "EC₅₀", i.e., the concentration for 50% of maximal effect, refers to a corresponding concentration of the antibody that induces 50% of the maximal effect.

The term "specific binding (or specifically bind)" used in this invention refers to a non-random binding reaction between two molecules, such as a reaction between an antibody and its targeted antigen. In certain embodiments, an antibody that specifically binds to a certain antigen (or an antibody specific to a certain antigen) refers to the antibody binding to the antigen with an affinity (KD) less than about 10⁻⁵ M, for example, less than about 10⁻⁶ M, 10⁻⁷ M, 10⁻⁸ M, 10⁻⁹ M, 10⁻¹⁰ M or less. In some embodiments of this invention, the term "targeting" refers to specific binding.

The term "KD" used in this invention refers to a dissociation equilibrium constant for a specific antibody-antigen interaction and is used for describing the binding affinity between an antibody and an antigen. A smaller equilibrium dissociation constant indicates tighter binding between an antibody and an antigen and a higher affinity between the antibody and the antigen. Generally, an antibody binds to an antigen with the equilibrium dissociation constant (KD) less than about 10⁻⁵ M, for example, less than about 10⁻⁶ M, 10⁻⁷ M, 10⁻⁸ M, 10⁻⁹ M, 10⁻¹⁰ M or less.

The term "single-chain variable fragment" or "scFv" refers to a fusion protein of a heavy chain variable region VH and a light chain variable region VL of an immunoglobulin, including different combination forms of VH at the N-terminus and VL at the N-terminus, which can be prepared by a conventional molecular cloning method for constructing recombinant proteins (Sambrook JF, E. F. et al., Molecular cloning: a laboratory manual. 4th ed., Cold Spring Harbor Laboratory Press, Cold Spring Harbor, New York: 2012).

The term "humanized antibody" refers to an antibody or antibody fragment obtained by replacing some or all of CDR regions of a humanized immunoglobulin (receptor antibody) with CDR regions of a non-humanized antibody (donor antibody), wherein the donor antibody may be a non-humanized (e.g., mouse, rat, or rabbit) antibody with the expected specificity, affinity, or reactivity. Furthermore, some amino acid residues in the framework region (FR) of the acceptor antibody may also be replaced with corresponding amino acid residues of the non-humanized antibody, or with amino acid residues of other antibodies, to further improve or optimize one or more characteristics of the antibody.

The term "host cell" refers to a cell into which an exogenous nucleic acid is introduced and its progeny, which can be transformed or transfected with a nucleotide encoding a polypeptide, to thereby express the exogenous polypeptide. The host cells described in this invention include, but are not limited to, CHO cells (Chinese hamster ovary cells), HEK293 cells (Human embryonic kidney cells 293), BHK cells (Baby Hamster Kidney cells), myeloma cells, yeast, insect cells, or prokaryotic cells such as *Escherichia coli.* It should be noted that the "host cell" described in this invention not only refers to a cell into which an exogenous nucleic acid is introduced, but also includes a progeny of the cell. The progeny cells may undergo mutations during cell division, but they still fall within the scope of the term as described in this invention.

As used herein, the term "effective amount" may refer to the amount of a composition described herein or a pharmaceutical formulation described herein, which causes a desired biological or medical response of tissues, systems, animals, plants, protozoa, bacteria, yeast, or humans, being sought by researchers, veterinarians, physicians, or other clinicians.

As used herein, the term "subject" refers to a vertebrate, or optionally refers to a mammal, or human. Mammals include, but are not limited to, murines, apes, humans, domestic animals, sport animals, and pets. Tissues, cells, and their progenies of bio-entities obtained *in vivo* or cultured *in vitro* are also included therein.

It should be understood that the terms used herein are merely for the purpose of describing the context such as embodiments and examples of this invention, and are not intended to limit the scope of this invention.

Some embodiments of this invention provide a B7-H3 antibody, its heavy chain complementarity determining regions (VH-CDR1, VH-CDR2, and VH-CDR3) of the B7-H3 antibody include amino acid sequences as set forth in SEQ ID Nos. 6-8, and light chain complementarity determining regions (VL-CDR1, VL-CDR2, and VL-CDR3) of the B7-H3 antibody include amino acid sequences as set forth in SEQ ID Nos. 14-16.

The B7-H3 antibody in this invention can bind to a B7-H3 antigen with a high affinity, can activate T cells, and has an important application value in the fields of immunotherapy and cancer treatment.

In some embodiments, the heavy chain variable regions of the B7-H3 antibody include an amino acid sequence as set forth in SEQ ID No. 1.

In some embodiments, the light chain variable regions of the B7-H3 antibody include an amino acid sequence as set forth in SEQ ID No. 9.
SEQ ID No. 1: 
QVQLVQSGAEVKKPGSSVKVSCKASGGTFSNYAINWVRQAPGQGLEWM
GGIIPLFGTANYAQKFQGRVTITADESTSTAYMELSSLRSDDTAVYYCARDQ
VVATSGVNFGMDVWGQGTTVTVSS.
SEQ ID No. 6 (VH-CDR1): GGTFSNYA.
SEQ ID No. 7 (VH-CDR2): IIPLFGTA.
SEQ ID No. 8 (VH-CDR3): ARDQWATSGVNFGMDV.
SEQ ID No. 9: 
DIQMTQEPSLTTSPGGTVTLTCRSSTGAVTTSNYANWVQEKPGQAPRGLI
GGTNKRAPGTPARFSGSLIGGKAALTITGVQPEDEAIYFCALWYSNLWVFG
GGTKLEIK.
SEQ ID No. 14 (VL-CDR1): TGAVTTSNY.
SEQ ID No. 15 (VL-CDR2): GTN.
SEQ ID No. 16 (VL-CDR3): ALWYSNLWV.

Some embodiments of this invention further provide a DNA fragment encoding the B7-H3 antibody described above.

In some embodiments, light chains of the B7-H3 antibody have a nucleotide sequence as set forth in SEQ ID NO. 50, and heavy chains of the B7-H3 antibody have a nucleotide sequence set forth in SEQ ID NO. 51.
SEQ ID NO. 50: 
gatattcagatgacacaagaaccaagtctcactacaagccctggaggcaccgttacactgacctgtcgc
tcttccactggcgccgtgaccacttccaactatgcaaactgggtgcaggagaaacctggacaggctcctag
aggtctgattggaggcactaacaagagagctccagggactcctgccaggttcagcggatctctgatcggtg
ggaaggcagctctgacaatcactggagtgcaacccgaggatgaggctatctacttctgtgctctctggtactc
aaatctgtgggtgttcggaggtggaacaaagctggagatcaag.
SEQ ID NO. 51:

caggtccagcttgtgcagtctggggctgaggtgaagaagcctgggtcctcggtgaaggtctcctgcaag
gcttctggaggcaccttcagcaactatgctatcaactgggtgcgacaggcccctggacaagggcttgagtg
gatgggagggatcatccctctctttggtacagcaaactacgcacagaagttccagggcagagtcacgatta
ccgcggacgaatccacgagcacagcctacatggagctgagcagcctgagatctgacgacacggccgtg
tattactgtgcgagagaccaggtagtggcaacgagtggcgtcaacttcggtatggacgtctggggccaagg
gaccacggtcaccgtctcctca.

Some embodiments of this invention further provide an expression vector containing at least one copy of the DNA fragment as described above.

Some embodiments of this invention further provide a host cell containing the expression vector as described above.

Some embodiments of this invention further provide an affinity-matured B7-H3 antibody derived from the B7-H3 antibody described above through mutation selection.

In some embodiments, the mutation selection includes random mutation of the light chain variable regions and/or heavy chain variable regions of the B7-H3 antibody. Without being bound by theory, it is believed that the mutation selection in this invention can further improve the affinity of the B7-H3 antibody.

In some embodiments, the heavy chain complementarity determining region CDR1 of the affinity-matured B7-H3 antibody includes an amino acid sequence as set forth in any one of SEQ ID No. 6 or SEQ ID Nos. 17-25, the heavy chain complementarity determining region CDR2 includes an amino acid sequence as set forth in SEQ ID No. 7 or any one of SEQ ID Nos. 26-37, and the heavy chain complementarity determining region CDR3 includes an amino acid sequence as set forth in SEQ ID No. 8 or any one of SEQ ID Nos. 38-48.

In some embodiments, the light chain complementarity determining regions of the affinity-matured B7-H3 antibody include amino acid sequences as set forth in SEQ ID Nos. 14-16.
SEQ ID No. 17: GGTLRPVL.
SEQ ID No. 18: GGTSGVAL.
SEQ ID No. 19: GGTRPAAL.
SEQ ID No. 20: GGTVRPAL.
SEQ ID No. 21: GGTGGIYL.
SEQ ID No. 22: GGTGGAYL.
SEQ ID No. 23: GGTSRPVL.
SEQ ID No. 24: GGTRPPAL.
SEQ ID No. 25: GGTTGRYL.
SEQ ID No. 26: IIPGFYSL.
SEQ ID No. 27: IIPMWQSV.
SEQ ID No. 28: IIPLFRSS.
SEQ ID No. 29: IIPRFGGV.
SEQ ID No. 30: IIPNFESV.
SEQ ID No. 31: IIPKFRSQ.
SEQ ID No. 32: IIPVFRSV.
SEQ ID No. 33: IIPGFDAV.
SEQ ID No. 34: IIPDFNSA.
SEQ ID No. 35: IIPRFASR.
SEQ ID No. 36: IIPNFHSS.
SEQ ID No. 37: IIPNFYSV.
SEQ ID No. 38: ARDRFTPRSGVNFGMDV.
SEQ ID No. 39: ARDREARESGVNFGMDV.
SEQ ID No. 40: ARDFDAPGSGVNFGMDV.
SEQ ID No. 41: ARDVWPRSGVNFGMDV.
SEQ ID No. 42: ARDIEVMASGVNFGMDV.
SEQ ID No. 43: ARDQVVPLSGVNFGMDV.
SEQ ID No. 44: ARDVNNPGSGVNFGMDV.
SEQ ID No. 45: ARDVEVFNSGVNFGMDV.
SEQ ID No. 46: ARDEWVGSGVNFGMDV.
SEQ ID No. 47: ARDAWPNSGVNFGMDV.
SEQ ID No. 48: ARDQVVPRSGVNFGMDV.

In some embodiments, the light chain variable regions of the affinity-matured B7-H3 antibody include the amino acid sequence as set forth in SEQ ID No. 9.

In some embodiments, the heavy chain framework regions of the affinity-matured B7-H3 antibody include amino acid sequences as set forth in SEQ ID Nos. 2-5.
SEQ ID No. 2: QVQLVQSGAEVKKPGSSVKVSCKAS.
SEQ ID No. 3: INWVRQAPGQGLEWMGG.
SEQ ID No. 4: NYAQKFQGRVTITADESTSTAYMELSSLRSDDTAVYYC.
SEQ ID No. 5: WGQGTTVTVSS.

In some embodiments, the light chain framework regions of the affinity-matured B7-H3 antibody include amino acid sequences as set forth in SEQ ID Nos. 10-13.
SEQ ID No. 10: DIQMTQEPSLTTSPGGTVTLTCRSS.
SEQ ID No. 11: ANWVQEKPGQAPRGLIG.
SEQ ID No. 12: KRAPGTPARFSGSLIGGKAALTITGVQPEDEAIYFC.
SEQ ID No. 13: FGGGTKLEIK.

Without being bound by theory, it is believed that the B7-H3 antibody and the affinity-matured B7-H3 antibody described above in this invention have the following properties:
a. specifically binding to human B7-H3 antigen;
b. specifically binding to cells overexpressing human B7-H3 membrane-proximal domain; and
c. mediating PBMC to kill B7-H3 positive tumors.

Some embodiments of this invention further provide a bispecific antibody. The bispecific antibody includes two heavy chains and two light chains. The heavy chains include an anti-B7-H3 heavy chain and an anti-CD3 heavy chain. The anti-B7-H3 heavy chain includes the heavy chain of the B7-H3 antibody described above or a heavy chain of the affinity-matured B7-H3 antibody described above. The light chains include an anti-B7-H3 light chain and an anti-CD3 light chain. The anti-B7-H3 light chain includes the light chain of the B7-H3 antibody described above or the light chain of the affinity-matured B7-H3 antibody described above.

In some embodiments, the anti-B7-H3 heavy chain binds to the anti-CD3 heavy chain through two disulfide bonds.

In some embodiments, a variable region of the anti-CD3 heavy chain includes an amino acid sequence as set forth in SEQ ID No. 49.

EVQLVESGGGLVQPGGSLKLSCAASGFTFNTYAMNWVRQAPGKGLEWV
ARIRSKYNNYATYYADSVKDRFTISRDDSKNTAYLQMNNLKTEDTAMYYCV
RHGNFGTSYVSWFAYWGQGTLVTVSS.

A variable region of the anti-CD3 light chain includes the amino acid sequence as set forth in SEQ ID No. 9.

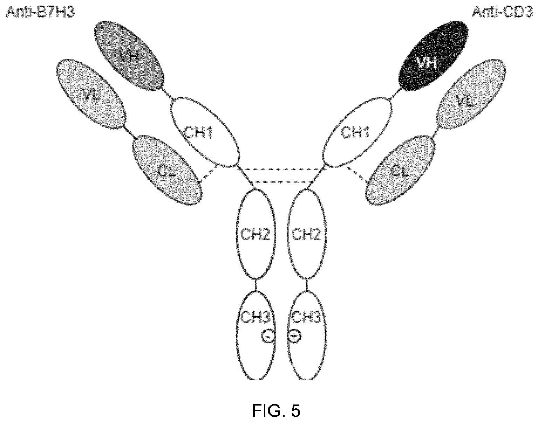

In some embodiments, structure of the bispecific antibody is shown in FIG. 5. The bispecific antibody includes an immunoglobulin Fab domain binding to B7-H3, an immunoglobulin Fab domain binding to CD3, and a heterodimeric Fc region. The immunoglobulin Fab domain binding to B7-H3 includes a variable region VH and a constant region CH1 of the anti-B7-H3 heavy chain as well as a variable region and constant region of the anti-B7-H3 light chain, and is capable of specifically binding to the B7-H3 antigen. The immunoglobulin Fab domain binding to the CD3 includes a variable region VH and a constant region CH1 of the anti-CD3 heavy chain as well as a variable region and constant region of the anti-CD3 light chain, and is capable of specifically binding to antigen CD3 on a surface of an immune cell. The heterodimeric Fc region contains an Fc fragment linked to the anti-B7-H3 heavy chain and an Fc fragment linked to the anti-CD3 heavy chain. The Fc fragment consists of the CH2 and CH3 constant domains.

In some embodiments, the Fc fragment linked to the anti-B7-H3 heavy chain contains a human Fc fragment or a humanized Fc fragment.

In some embodiments, the Fc fragment linked to the anti-B7-H3 heavy chain contains a human IgG1 Fc fragment.

In some embodiments, the Fc fragment linked to the anti-CD3 heavy chain contains a human Fc fragment or a humanized Fc fragment.

In some embodiments, the Fc fragment linked to the anti-CD3 heavy chain contains a human IgG1 Fc fragment.

In some embodiments, the CH3 of the Fc fragment linked to the anti-B7-H3 heavy chain contains T394D, P395D, and P396D mutations.

In some embodiments, the CH3 of the Fc fragment linked to the anti-CD3 heavy chain contains P395K, P396K, and V397K mutations.

In some embodiments, the CH2 of the Fc fragment linked to the anti-B7-H3 heavy chain contains L234A or L235A, and P329G mutations.

The bispecific antibody provided in some embodiments of this invention have the following properties:
a. binding to the B7-H3 and the CD3;
b. functioning to activate T cells;
c. mediating the T cells to kill B7-H3 positive tumor cells; and
d. inhibiting growth of B7-H3 positive tumors *in vivo.*

This invention further provides a biomaterial, which is any one of the following B1) to B6):
B1) a nucleic acid molecule encoding a heavy chain and/or a light chain of the antibody of any one of (b1) to (b3), or a nucleic acid molecule encoding a heavy chain and/or a light chain in the antigen-binding domain of the antibody of any one of (b1) to (b3): (b1) is the B7-H3 antibody in the above, (b2) is any one of the affinity-matured B7-H3 antibodies described above, or (b3) is any one of the bispecific antibodies described above;
B2) an expression cassette containing the nucleic acid molecule described in B1);
B3) a recombinant vector containing the nucleic acid molecule described in B1), or a recombinant vector containing the expression cassette described in B2);
B4) a recombinant microorganism containing the nucleic acid molecule described in B1), or a recombinant microorganism containing the expression cassette described in B2), or a recombinant microorganism containing the recombinant vector described in B3);
B5) a cell line containing the nucleic acid molecule described in B1), or a cell line containing the expression cassette described in B2), or a cell line containing the recombinant vector described in B3); and
B6) a nucleic acid molecule encoding the variable region of heavy chain and/or light chain of the antibody of any one of (b1) to (b3), or a nucleic acid molecule encoding the variable region of heavy chain and/or the variable region of light chain in the antigen-binding domain of the antibody of any one of (b1) to (b3).

Some embodiments of this invention provide a pharmaceutical composition. The pharmaceutical composition includes any one or a combination of at least two of the B7-H3 antibody described above, the affinity-matured B7-H3 antibody described above, or the bispecific antibody described above.

In some embodiments, the pharmaceutical composition further includes any one or a combination of at least two of a pharmaceutically acceptable carrier, a diluent, or an excipient.

In some embodiments, the present disclosure relates to a pharmaceutical composition, which contains the antibody or antibody fragment, the bispecific antibody or an antibody conjugate in the present disclosure, and optionally a pharmaceutically acceptable carrier, a surfactant, and/or a diluent. In some embodiments, in addition to the antibody, the bispecific antibody or the antibody conjugate in the present disclosure, the pharmaceutical composition further contains one or more additional therapeutic agents. In some embodiments, the additional therapeutic agents include, but are not limited to, chemotherapeutic agents, growth inhibitors, cytotoxic agents, reagents for radiotherapy, antiangiogenic agents, apoptotic agents, anti-tubulin agents, and others reagents for treating cancer.

Some embodiments of this invention further provide use of the B7-H3 antibody, the affinity-matured B7-H3 antibody, the bispecific antibody described above or the pharmaceutical composition described above in the preparation of an anti-tumor drug.

Some embodiments of this invention further provide use of the B7-H3 antibody, the affinity-matured B7-H3 antibody described in any one of the above, the bispecific antibody described in any one of the above or the pharmaceutical composition in tumor treatment.

Some embodiments of this invention further provide a method for treating tumor-associated diseases, wherein the B7-H3 antibody, the affinity-matured B7-H3 antibody described in any one of the above, the bispecific antibody described in any one of the above or the pharmaceutical composition is administered to a subject in need thereof. In some embodiments, the tumor includes any one or a combination of at least two of neurological system tumor, colorectal cancer, liver cancer, head and neck cancer, lung cancer, melanoma, pancreatic cancer, gastric cancer, kidney cancer, bladder cancer, breast cancer, ovarian cancer or prostate cancer.

The present disclosure provides a B7-H3 antibody and use thereof. The B7-H3 antibody binds to a B7-H3 antigen with a high affinity, has an important application value in the fields of immunotherapy and cancer treatment, overcomes the limitations of the prior art, and satisfies practical requirements.
(1) The B7-H3 antibody in this invention can bind to B7-H3 antigen and/or cells expressing B7-H3 antigen, with a high affinity, and can efficiently mediate PBMC to destroy B7-H3 positive tumors.
(2) In this invention, the affinity of the B7-H3 antibody is further improved by mutating the complementarity determining regions of the heavy chain of the B7-H3 antibody.
(3) In this invention, B7-H3×CD3 bispecific antibody is constructed using the B7-H3 antibody. The bispecific antibody has high affinity and stability, can effectively mediate PBMC to kill tumor cells, and has a significant effect in tumor growth inhibition.

This invention further encompasses nucleic acid sequences encoding these polypeptide chains. In a process of expressing antibodies, nucleic acid sequences are inserted into suitable vectors, the vectors including but not limited to: plasmids, phage expression vectors, cosmids, artificial chromosomes, phagesand animal viruses. The expression vectors contain elements for regulating expression, including, but not limited to, promoters, transcription initiation sequences, enhancers, signal peptide sequences, etc. The promoters include, but are not limited to, T7 promoters, T3 promoters, SP6 promoters, β-actin promoters, EF-1α promoters, CMV promoters, and SV40 promoters. The expression vectors may be transferred into host cells using an appropriate method known in the art, including but not limited to: a calcium phosphate precipitation method, a polyethylene imine transfection method, a liposome transfection method, an electroporation method, and a PEI (polyethylene imine) transfection method.

### Examples

### Example 1

The present example provides a preparation method of B7-H3 antibody. The method includes the following steps:
(1) The recombinant antigen of the extracellular domain (ECD) (29-461) of B7-H3 was prepared, with sequence derived from UniProtKB: Q5ZPR3 (CD276_HUMAN). The antigen gene was cloned into the eukaryotic expression vector pcDNA3.1-TEV-cFc-His, and hB7-H3-TEV-cFc-His recombinant eukaryotic expression plasmid was established. Then the plasmid was transfected into 293F cells using PEI. After five days of culture, the cell supernatant was collected, subjected to protein A affinity purification to obtain a B7-H3-TEV-cFc-His fusion protein, and then enzymatic cleavage by using TEV protease, and purified by a nickel column to obtain human B7-H3 antigen.
(2) PBMC was isolated from human peripheral blood. Total RNAs were extracted from the isolated PBMC, and cDNAs were synthesized using a reverse transcription kit. The genes of human immunoglobulin heavy chain variable domain were obtained by specific PCR amplification to amplify VH fragments thereof, using the cDNAs as templates and using designed specific primers according to Lim. T. S., et al (Lim T S, Mollova S, Rubelt F, et al. An optimised procedure for amplification of rearranged human antibody genes of different isotypes[J]. New Biotechnology, 2010, 27(2):108-117.).
(3) The light chain sequence (SEQ ID No: 9) of B7-H3 antibody was derived from an anti-CD3 antibody, which was described in a published patent (CN110551221A) by the inventors. The genes of light chain variable domain (VL) of anti-CD3 antibody were obtained by PCR amplification using designed specific primers;
(4) The human VH genes and the VL genes was assembled into a scFv fragment with a G4S-linker using the overlap extension PCR technique. These fragments were cloned into phagemids, and then transformed in TG1 electrocompetent cells (Lucigen) by electroporation to obtain a phagemid library of the human-VH: CD3-VL scFvs. The scFv-displaying phage were rescued with helper phage and prepared (PEG precipitation) for later use. An appropriate amount of recombinant human B7-H3 antigens was added to each well of a 96-well plate for incubation overnight. After incubation, the plate was washed several times with PBST (a phosphate buffer solution containing Tween-20), and then the scFv-displaying phage was added for panning and screening. After several rounds of screening and sequencing of the enriched positive clones, a B7-H3 antibody was obtained and named 5D9. Amino acid sequences of the heavy chain variable region (VH) and light chain variable region (VL) of 5D9 are shown in Table 1.

**Table 1**

| Na me | Region | Sequence | SE Q ID No |
|---|---|---|---|
| 5D9 | Heavy chain variable region (VH) | QVQLVQSGAEVKKPGSSVKVSCKASGGTFSNYAINWVRQAP GQGLEWMGGIIPLFGTANYAQKFQGRVTITADESTSTAYME LSSLRSDDTAVYYCARDQVVATSGVNFGMDVWGQGTTVTV SS | 1 |
| | VH-FR1 | QVQLVQSGAEVKKPGSSVKVSCKAS | 2 |
| | VH-FR2 | INWVRQAPGQGLEWMGG | 3 |
| | VH-FR3 | NYAQKFQGRVTITADESTSTAYMELSSLRSDDTAVYYC | 4 |
| | VH-FR4 | WGQGTTVTVSS | 5 |
| | VH-CDR1 | GGTFSNYA | 6 |
| | VH-CDR2 | IIPLFGTA | 7 |
| | VH-CDR3 | ARDQVVATSGVNFGMDV | 8 |
| | Light chain variable region (VL) | DIQMTQEPSLTTSPGGTVTLTCRSSTGAVTTSNYANWVQEK PGQAPRGLIGGTNKRAPGTPARFSGSLIGGKAALTITGVQPE DEAIYFCALWYSNLWVFGGGTKLEIK | 9 |
| | VL-FR1 | DIQMTQEPSLTTSPGGTVTLTCRSS | 10 |
| | VL-FR2 | ANWVQEKPGQAPRGLIG | 11 |
| | VL-FR3 | KRAPGTPARFSGSLIGGKAALTITGVQPEDEAIYFC | 12 |
| | VL-FR4 | FGGGTKLEIK | 13 |
| | VL-CDR1 | TGAVTTSNY | 14 |
| | VL-CDR2 | GTN | 15 |
| | VL-CDR3 | ALWYSNLWV | 16 |

### Example 2

In this example, the antibody 5D9 described in Example 1 was expressed, purified, and analyzed.

All antibodies in this invention were transiently expressed by 293F cells. The gene encoding heavy chain and light chain of the 5D9 antibody were synthesized respectively, which were cloned into expression vectors pcDNA3.1. These plasmids were transfected into the 293F cells using PEI. After six days of culture, the supernatant was collected by centrifugation, and subsequently purified using a protein A affinity chromatography column (GE Healthcare).

The sequences of the positive control were derived from WO2011US26689 (Enoblituzumab) and WO2003US07004 (8H9). The positive controls were expressed and purified by the method described above.

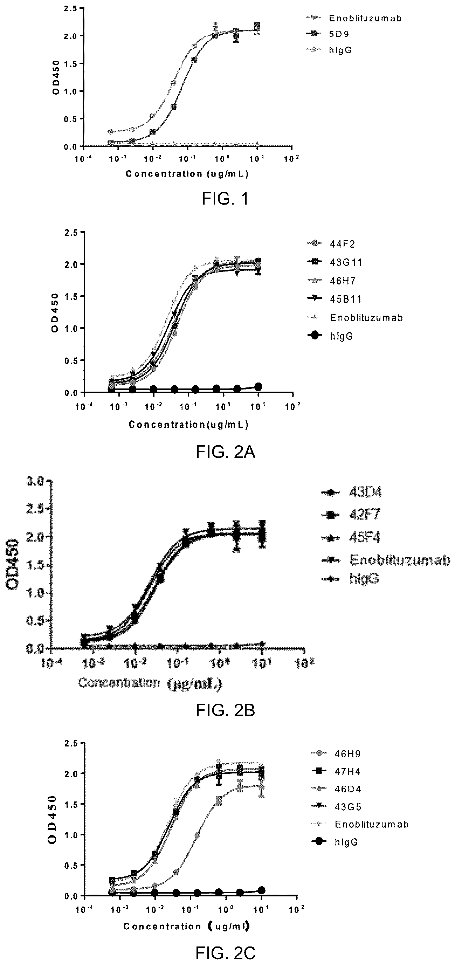

The binding activity of the 5D9 to B7-H3 antigen was analyzed by ELISA. Human B7-H3 antigens were coated on the 96-well plate and placed at 4 °C overnight. The antibody 5D9 and controls were diluted to 10 µg/mL, followed by 4-fold dilution gradients to generate a total of 8 different concentrations. The diluted samples and the wells coated antigen were incubated for 1h. After washing, HRP (horseradish peroxidase)-labeled goat anti-human IgG (H+L) secondary antibody was added to the plate and incubated for 1h. TMB (3,3',5,5'-tetramethylbenzidine) solution was then added to each well after washing for chromogenesis. The absorbance was measured at OD₄₅₀ₙₘ with a microplate reader. Taking the logarithmic value of the sample concentration as the X-axis, and the absorbance OD₄₅₀ₙₘ value as the Y-axis, a dose-response curve was drawn to calculate the EC₅₀. The EC₅₀ represents the binding activity of the antibody to B7-H3 antigen. A negative control IgG group and a positive antibody Enoblituzumab control group were meanwhile set in the experiment. Results are shown in FIG. 1.

As shown in FIG. 1, 5D9 exhibits a good binding activity to B7-H3 antigen, which is slightly weaker than that of Enoblituzumab to B7-H3 antigen.

### Example 3

The present example discloses an affinity-matured variants of the B7-H3 antibody.

As can be seen from the foregoing description, the B7-H3 antibody 5D9 obtained in the present disclosure has a slightly weaker binding activity to the B7-H3 antigen than the Enoblituzumab. To further obtain an antibody with a higher binding affinity to B7-H3 antigen, the affinity-matured B7-H3 antibody was obtained by performing *in vitro* affinity maturation on variable regions of a heavy chain of the antibody 5D9. A preparation method included the following steps:
(1) To generate an affinity-matured library, the upstream and downstream random primers were synthesized using randomized triple codons of the heavy chain CDRs (HC-CDR1, HC-CDR2, and HC-CDR3) of the 5D9 antibody. Random mutation genes of the heavy chain CDR of the 5D9 were obtained by SOE-PCR. Following the cloning step, the phagemid vectors containing the mutated genes were transformed into TG1 electrocompetent cells (Lucigen), to obtain a random mutation phage library of the heavy chain complementarity determining regions of the 5D9.
(2) Panning and screening of the affinity-matured libraries: the protocol of phage preparation and screening was referred as above described. After several rounds of panning, enriching positive clones and sequencing, 33 affinity-matured variants were obtained. The light chain variable region (VL) of these 33 affinity-matured variants had an amino acid sequence as set forth in SEQ ID No. 9, and the heavy chain framework regions VH-FR1, VH-FR2, VH-FR3, and VH-FR4 had amino acid sequences as set forth in SEQ ID No. 2, SEQ ID No. 3, SEQ ID No. 4, and SEQ ID No. 5, respectively.

The amino acid sequences of the heavy chain complementarity determining regions VH-CDR1, VH-CDR2, and VH-CDR3 of various variants are set forth in Table 2.

**Table 2**

| Name | Region | Sequence | SEQ ID No: |
|---|---|---|---|
| 44F2 | VH-CDR1 | GGTFSNYA | 6 |
| | VH-CDR2 | IIPGFYSL | 26 |
| | VH-CDR3 | ARDQVVATSGVNFGMDV | 8 |
| 43G11 | VH-CDR1 | GGTLRPVL | 17 |
| | VH-CDR2 | IIPLFGTA | 7 |
| | VH-CDR3 | ARDQVVATSGVNFGMDV | 8 |
| 46H7 | VH-CDR1 | GGTFSNYA | 6 |
| | VH-CDR2 | IIPLFGTA | 7 |
| | VH-CDR3 | ARDRFTPRSGVNFGMDV | 38 |
| 45B11 | VH-CDR1 | GGTFSNYA | 6 |
| | VH-CDR2 | IIPMWQSV | 27 |
| | VH-CDR3 | ARDQVVATSGVNFGMDV | 8 |
| 43D4 | VH-CDR1 | GGTSGVAL | 18 |
| | VH-CDR2 | IIPLFGTA | 7 |
| | VH-CDR3 | ARDQVVATSGVNFGMDV | 8 |
| 42F7 | VH-CDR1 | GGTRPAAL | 19 |
| | VH-CDR2 | IIPLFGTA | 7 |
| | VH-CDR3 | ARDQVVATSGVNFGMDV | 8 |
| 45F4 | VH-CDR1 | GGTFSNYA | 6 |
| | VH-CDR2 | IIPLFRSS | 28 |
| | VH-CDR3 | ARDQVVATSGVNFGMDV | 8 |
| 46H9 | VH-CDR1 | GGTFSNYA | 6 |
| | VH-CDR2 | IIPLFGTA | 7 |
| | VH-CDR3 | ARDREARESGVNFGMDV | 39 |
| 47H4 | VH-CDR1 | GGTFSNYA | 6 |
| | VH-CDR2 | IIPLFGTA | 7 |
| | VH-CDR3 | ARDFDAPGSGVNFGMDV | 40 |
| 46D4 | VH-CDR1 | GGTFSNYA | 6 |
| | VH-CDR2 | IIPLFGTA | 7 |
| | VH-CDR3 | ARDVVVPRSGVNFGMDV | 41 |
| 43G5 | VH-CDR1 | GGTVRPAL | 20 |
| | VH-CDR2 | IIPLFGTA | 7 |
| | VH-CDR3 | ARDQVVATSGVNFGMDV | 8 |
| 44C5 | VH-CDR1 | GGTFSNYA | 6 |
| | VH-CDR2 | IIPRFGGV | 29 |
| | VH-CDR3 | ARDQVVATSGVNFGMDV | 8 |
| 42A1 | VH-CDR1 | GGTGGIYL | 21 |
| | VH-CDR2 | IIPLFGTA | 7 |
| | VH-CDR3 | ARDQVVATSGVNFGMDV | 8 |
| 47C8 | VH-CDR1 | GGTFSNYA | 6 |
| | VH-CDR2 | IIPLFGTA | 7 |
| | VH-CDR3 | ARDIEVMASGVNFGMDV | 42 |
| 48E9 | VH-CDR1 | GGTGGAYL | 22 |
| | VH-CDR2 | IIPLFGTA | 7 |
| | VH-CDR3 | ARDQVVATSGVNFGMDV | 8 |
| 49C3 | VH-CDR1 | GGTFSNYA | 6 |
| | VH-CDR2 | IIPNFESV | 30 |
| | VH-CDR3 | ARDQVVATSGVNFGMDV | 8 |
| 49C8 | VH-CDR1 | GGTFSNYA | 6 |
| | VH-CDR2 | IIPKFRSQ | 31 |
| | VH-CDR3 | ARDQVVATSGVNFGMDV | 8 |
| 49E1 | VH-CDR1 | GGTFSNYA | 6 |
| | VH-CDR2 | IIPVFRSV | 32 |
| | VH-CDR3 | ARDQVVATSGVNFGMDV | 8 |
| 49F6 | VH-CDR1 | GGTFSNYA | 6 |
| | VH-CDR2 | IIPGFDAV | 33 |
| | VH-CDR3 | ARDQVVATSGVNFGMDV | 8 |
| 50A1 | VH-CDR1 | GGTFSNYA | 6 |
| | VH-CDR2 | IIPLFGTA | 7 |
| | VH-CDR3 | ARDQVVPLSGVNFGMDV | 43 |
| 50E3 | VH-CDR1 | GGTFSNYA | 6 |
| | VH-CDR2 | IIPLFGTA | 7 |
| | VH-CDR3 | ARDVNNPGSGVNFGMDV | 44 |
| 49H5 | VH-CDR1 | GGTFSNYA | 6 |
| | VH-CDR2 | IIPDFNSA | 34 |
| | VH-CDR3 | ARDQVVATSGVNFGMDV | 8 |
| 50B10 | VH-CDR1 | GGTFSNYA | 6 |
| | VH-CDR2 | IIPLFGTA | 7 |
| | VH-CDR3 | ARDVEVFNSGVNFGMDV | 45 |
| 48G2 | VH-CDR1 | GGTSRPVL | 23 |
| | VH-CDR2 | IIPLFGTA | 7 |
| | VH-CDR3 | ARDQVVATSGVNFGMDV | 8 |
| 49D 12 | VH-CDR1 | GGTFSNYA | 6 |
| | VH-CDR2 | IIPRFASR | 35 |
| | VH-CDR3 | ARDQVVATSGVNFGMDV | 8 |
| 48D10 | VH-CDR1 | GGTRPAAL | 19 |
| | VH-CDR2 | IIPLFGTA | 7 |
| | VH-CDR3 | ARDQVVATSGVNFGMDV | 8 |
| 48A6 | VH-CDR1 | GGTRPPAL | 24 |
| | VH-CDR2 | IIPLFGTA | 7 |
| | VH-CDR3 | ARDQVVATSGVNFGMDV | 8 |
| 48B 12 | VH-CDR1 | GGTTGRYL | 25 |
| | VH-CDR2 | IIPLFGTA | 7 |
| | VH-CDR3 | ARDQVVATSGVNFGMDV | 8 |
| 49B2 | VH-CDR1 | GGTFSNYA | 6 |
| | VH-CDR2 | IIPNFHSS | 36 |
| | VH-CDR3 | ARDQVVATSGVNFGMDV | 8 |
| 49C7 | VH-CDR1 | GGTFSNYA | 6 |
| | VH-CDR2 | IIPNFYSV | 37 |
| | VH-CDR3 | ARDQVVATSGVNFGMDV | 8 |
| 50A4 | VH-CDR1 | GGTFSNYA | 6 |
| | VH-CDR2 | IIPLFGTA | 7 |
| | VH-CDR3 | ARDEVVVGSGVNFGMDV | 46 |
| 50A6 | VH-CDR1 | GGTFSNYA | 6 |
| | VH-CDR2 | IIPLFGTA | 7 |
| | VH-CDR3 | ARDAVVPNSGVNFGMDV | 47 |
| 50A11 | VH-CDR1 | GGTFSNYA | 6 |
| | VH-CDR2 | IIPLFGTA | 7 |
| | VH-CDR3 | ARDQVVPRSGVNFGMDV | 48 |

### Example 4

The affinity-matured B7-H3 antibodies in Example 3 were expressed and purified in this example.

According to the method described in Example 2, the heavy chain and light chain DNAs encoding the variants of affinity-matured antibodies prepared in Example 4 were respectively synthesized, cloned into expression vectors pcDNA3.1 and transfected into 293F cells by PEI. After 5-6 days of culture, the supernatant was collected and purified by Protein A affinity chromatography column (GE Healthcare). 33 affinity-matured B7-H3 antibodies were obtained.

### Example 5

In this example, the functional verification of the affinity-matured variants obtained in Example 4 was carried out.

### (1) The binding activity of affinity-matured variants to human B7-H3 antigen was analyzed by ELISA

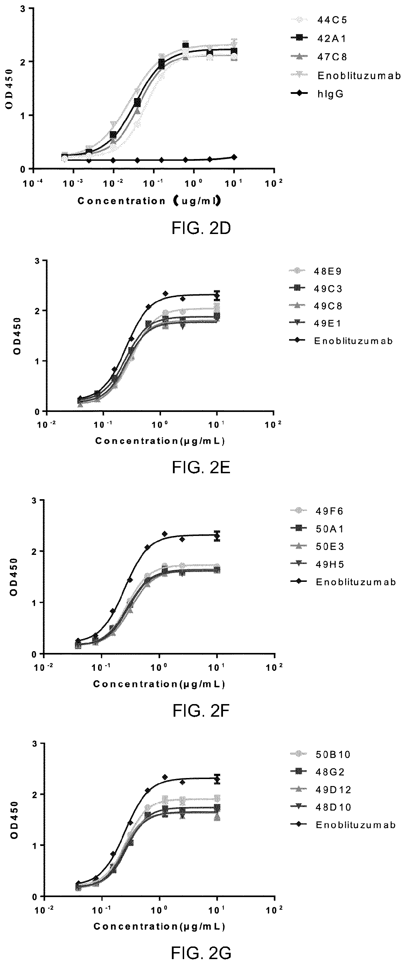

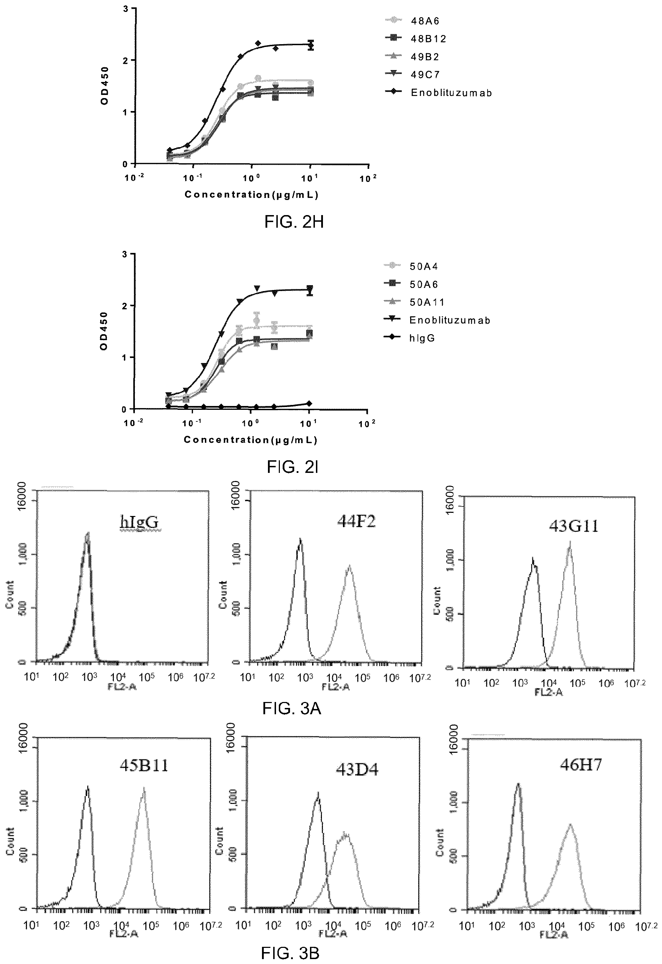

ELISA was used to analyze the binding activity of affinity-matured variants to human B7-H3 antigen. By taking the logarithmic value of the sample concentration as the X-axis, and the absorbance OD₄₅₀ₙₘ value as the Y-axis, a dose-response curve was drawn to calculate the EC₅₀. The EC₅₀ represents the binding activity of the affinity-matured variants to B7-H3 antigen. A positive control Enoblituzumab and a negative control hlgG were set in the experiment. Results are shown in FIG. 2A to FIG. 2I.

As shown in FIG. 2A to FIG. 2I, the affinity-matured variants have relatively higher binding activities to human B7-H3 antigen, most of which are similar to that of Enoblituzumab. The EC₅₀ values of the affinity-matured variants are shown in Table 3.

**Table 3**

| Name | EC₅₀ ( µg/mL ) | Name | EC₅₀ ( µg/mL ) | Name | EC₅₀ ( µg/mL ) |
|---|---|---|---|---|---|
| 44F2 | 0.04642 | 42A1 | 0.03536 | 49D 12 | 0.02187 |
| 43G11 | 0.04114 | 47C8 | 0.04260 | 48D10 | 0.02511 |
| 46H7 | 0.03902 | 48E9 | 0.04381 | 48A6 | 0.02723 |
| 45B11 | 0.02649 | 49C3 | 0.02447 | 48B12 | 0.02587 |
| 43D4 | 0.03029 | 49C8 | 0.02848 | 49B2 | 0.02864 |
| 42F7 | 0.02777 | 49E1 | 0.02614 | 49C7 | 0.03303 |
| 45F4 | 0.02191 | 49F6 | 0.03088 | 50A4 | 0.02742 |
| 46H9 | 0.1470 | 50A1 | 0.03212 | 50A6 | 0.02466 |
| 47H4 | 0.02435 | 50E3 | 0.04175 | 50A11 | 0.03390 |
| 46D4 | 0.02827 | 49H5 | 0.03357 | Enoblituzumab | 0.02551 |
| 43G5 | 0.02790 | 50B10 | 0.02778 | | |
| 44C5 | 0.06389 | 48G2 | 0.03032 | | |

### (2) Analysis of binding activity of affinity-matured variants to the overexpressing cells of B7-H3 membrane-proximal domain by flow cytometry (FACS)

In order to further investigate the binding activity of the affinity-matured variants with functional regions of human B7-H3, their binding activity to the overexpressing cells of the B7-H3 membrane-proximal domain was detected by FACS. First, a stable cell line that expresses B7-H3 membrane-proximal domain (243-534) was constructed as positive cells (Positive), and same original cells not expressing the B7-H3 membrane-proximal domain as a blank (negative cells, Negative).

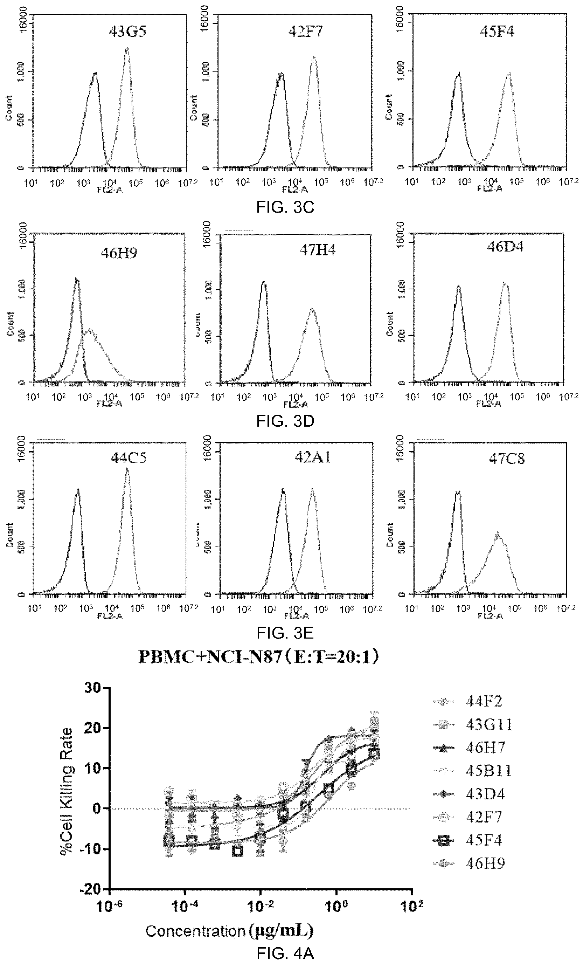

Both positive cells and negative cells were collected, then washed once with 2% FBS/PBS diluent pre-cooled at 4 °C. Afterwards, the cell was resuspended in the same buffer to achieve a cell density of 5.0×10⁶ cells/mL. The cells was inoculated onto a 96-well plate at a volume of 100 µL/well. Each affinity-matured variant was prepared with diluent at a concentration of 5 µg/mL. 100 µL of antibody solutions were mixed with an equal volume of positive cells and negative cells. After incubated at 4 °C for 1 h, the 96-well plate was centrifuged at 500×g with 4°C for 5 min to remove supernatant. The cells were washed twice and resuspended with PE anti-human IgG Fc (Invitrogen) diluent, incubated at 4 °C for 1h in the dark. Supernatant was removed by centrifugion at 500×g with 4°C for 5 min. Cells were resuspended with 200 µL of diluent after twice washing, and then immediately analyzed by CytoFLEX. Results are shown in FIG. 3A to FIG. 3E.

The results are shown in FIG. 3A to FIG. 3E. All of the affinity-matured variants can bind to the positive cells expressing the B7-H3 membrane-proximal domain (solid lines on the right of the drawings), but do not bind to the negative cells (solid lines on the left of the drawings).

### (3) PBMC-mediated cytotoxicity against B7-H3 positive tumor cells in vitro of affinity-matured variants.

According to the foregoing ELISA and FACS results, 15 affinity-matured variants with higher affinity to the human B7-H3 antigen (44F2, 43G11, 46H7, 45B11, 43D4, 42F7, 45F4, 46H9, 46D4, 47H4, 48E9, 49C3, 49E1, 50A1, and 50A6) were selected to analyze the T-cell-mediated-cytotoxic activity. In this study, human gastric cancer NCI-N87 cells (B7-H3 positive) were used as target cells and human PBMCs were used as effector cells.

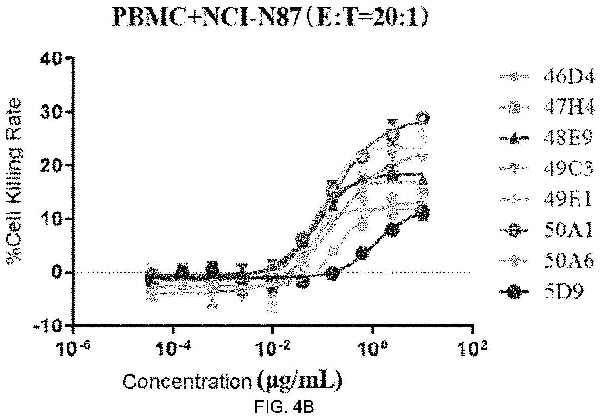

The NCI-N87 cells were digested with pancreatin, to prepare single-cell suspension, and the cell density was adjusted to 0.40×10⁶ cells/mL with phenol red-free 5% FBS-RPMI 1640 culture medium, and 50 µL/well was added to a 96-well plate. The human PBMCs were adjusted to 4.00×10⁶ cells/mL, and 100 µL/well was added. Antibodies were diluted to 40 µg/mL with the phenol red-free 5% FBS-RPMI 1640 culture medium, and then subjected to a four-fold serial dilution to obtain ten concentrations, i.e., 40 µg/mL, 10 µg/mL, 2.5 µg/mL, 0.625 µg/mL, 0.1563 µg/mL, 0.0391 µg/mL, 0.0098 µg/mL, 0.0024 µg/mL, 0.0006 µg/mL, and 0.00015 µg/mL, respectively. The diluted antibodies were added to the 96-well plate at 50 µL/well. The 96-well plate was placed in an incubator at 37 °C with 5% CO₂ overnight. The next day (about 20 hours later), cellular cytotoxicity activity was detected using a lactate dehydrogenase cytotoxicity kit (Beyotime). The cytotoxicity rate was calculated as Cytotoxicity%=( sample OD value -SR)/(MR-SR)×100%, where SR= spontaneous release wells OD value (target cells + effector cells), MR= maximum release wells OD value (target cells). The results are shown in FIG. 4A and FIG. 4B.

As shown in FIG. 4A and FIG. 4B, each of the 15 various affinity-matured variants can mediate the killing of PBMCs to B7-H3 positive human gastric cancer NCI-N87 cells.

### Example 6

The present example provides bispecific antibodies targeting CD3 and B7-H3.

### (1) Design of B7-H3×CD3 bispecific antibody

The affinity-matured B7-H3 antibodies (42F7, 43G11, 49E1, and 46H7) previously obtained were chosen to develop bispecific antibodies targeting CD3 and B7-H3. The bispecific antibodies in the examples include two different heavy chains and share a common light chain. A structural schematic diagram is shown in FIG. 5, containing four polypeptide chains, respectively named as anti-B7-H3 heavy chain (VH-CH1-hinge-CH2-CH3- in sequence from N-terminus to C-terminus), anti-CD3 heavy chain (VH-CH1-hinge-CH2-CH3- in sequence from N-terminus to C-terminus), anti-B7-H3 light chain (VL-CL in sequence from N-terminus to C-terminus), and anti-CD3 light chain (VL-CL in sequence from N-terminus to C-terminus), wherein the anti-B7-H3 light chain and the anti-CD3 light chain are a common light chain, and the CH3 domains of the two heavy chains contain asymmetric amino acids modifications with opposite charges, to form the heterodimeric Fc region described above. These four polypeptide chains formed a structural with an immunoglobulin Fab domain specifically binding to B7-H3, an immunoglobulin Fab domain specifically binding to CD3, and a heterodimeric Fc region.

The immunoglobulin Fab domain specifically binding to B7-H3, containing the heavy chain and the light chain of anti-B7-H3, the sequences is derived from the affinity-matured B7-H3 antibodies (42F7, 43G11, 49E1, and 46H7) in the foregoing examples.

The immunoglobulin Fab domain specifically binding to CD3 containing the heavy chain(SEQ ID No. 49) and the light chain of anti-CD3 (SEQ ID No. 9).
SEQ ID No. 49: 
EVQLVESGGGLVQPGGSLKLSCAASGFTFNTYAMNWVRQAPGKGLEWV
ARIRSKYNNYATYYADSVKDRFTISRDDSKNTAYLQMNNLKTEDTAMYYCV
RHGNFGTSYVSWFAYWGQGTLVTVSS.
SEQ ID No. 9:

DIQMTQEPSLTTSPGGTVTLTCRSSTGAVTTSNYANWVQEKPGQAPRGLI
GGTNKRAPGTPARFSGSLIGGKAALTITGVQPEDEAIYFCALWYSNLWVFG
GGTKLEIK.

The modification of Fc domain was described in published PCT (WO2017034770A1) patent. The CH3 domain of the anti-B7-H3 heavy chain had the following mutations: T394D, P395D, and P396D (EU), named OB, with negative charge; The CH3 domain of the anti-CD3 heavy chain had the following mutations: P395K, P396K, and V397K (EU), named OA, with positive charge. To minimize the binding activity between the Fc domain and FcγR, the CH2 domains also contain L234A, L235A, and P329G (LALA-PG) mutations.

### (2) Construction of expression plasmids

The gene encoding the variants of affinity-matured antibodies (42F7, 43G11, 49E1, and 46H7) were cloned into the eukaryotic expression vector pFUSEss-CHIg-hG1-Fc1 (containing OB, L234A, L235A, and P329G mutations), named as plasmid 1. The anti-CD3 heavy chain was cloned into the eukaryotic expression vector pFUSE-hlgG1-Fc2 (containing OA, L234A, L235A, and P329G mutations), named as plasmid 2. The gene of anti-B7-H3/anti-CD3 light chain was cloned into pCDNA3.1, named as plasmid 3.

### (3) Expression and purification

The recombined plasmid 1, plasmid 2, and plasmid 3 were prepared, and cotransfected with HEK293F cells by PEI. Suspension was placed in a culture shaker under 37 °C, 5% CO₂, and 120 rpm to be cultured for 6 days in the dark. The culture supernatant was centrifuged and purified by protein A affinity chromatography. The purified bispecific antibodies were analyzed by SDS-PAGE. These B7-H3×CD3 bispecific antibodies were named as: 42F7×CD3, 43G11×CD3, 49E1×CD3, and 46H7×CD3. A positive control named 8H9×CD3 also prepared by the same method.

### Example 7

In this example, the functional verification of B7-H3×CD3 bispecific antibodies described in Example 6 was carried out.

### (1) Binding activity of the B7-H3×CD3 bispecific antibodies

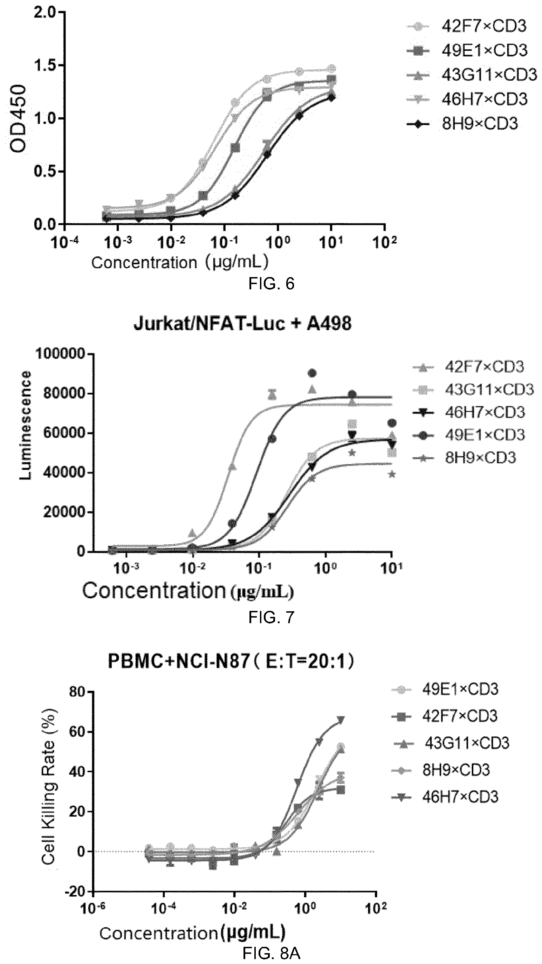

The binding activity of the B7-H3×CD3 bispecific antibodies to B7-H3 was analyzed by ELISA (refers to Example 2). The method included: adding B7-H3 antigen diluted to 1 µg/mL to a microtiter plate at 100 µL/well, and coating overnight at 4 °C, and the next day, washing with 0.05% PBST, then blocking for 2 h at a room temperature, washing again with 0.05% PBST, then adding serial diluted B7-H3×CD3 bispecific antibodies (diluted in PBS), incubating at a room temperature for 1 h, washing the unbound with 0.05% PBST, subsequently adding HRP-labeled goat anti-human IgG (H+L) secondary antibody (Proteintech, Cat.SA0001-17), incubating at a room temperature for 1 h, washing with 0.05% PBST, adding a TMB chromogenic solution, incubating at a room temperature for 5 min in a dark place, terminating reaction using H₂SO₄, detecting absorbance at 450 nm using a microplate reader, and drawing dose-effect curves with log (antibody concentration) as an abscissa and the absorbance OD₄₅₀ as an ordinate. The results are shown in FIG. 6. As demonstrated from FIG. 6, each of B7-H3×CD3 bispecific antibodies has high binding affinity to human B7-H3.

### (2) The activation of T cells by B7-H3×CD3 bispecific antibodies

To evaluate the activation of T cells by B7-H3×CD3 bispecific antibodies, an Jurkat-NFAT-luciferase assay was employed. This assay consisting of the Jurkat/NFAT-Luc reporter gene cell line and B7-H3 positive human kidney cancer A498 cells. In the Jurkat/NFAT-Luc cell line, luciferase gene was regulated by NFAT (Nuclear factor of activated T-cell) transcription factor, acted as effector cells, and B7-H3 positive A498 cells acted as target cells. When coculturing Jurkat/NFAT-Luc cells with A498 cells, there is no expression of luciferase. However, the B7-H3×CD3 bispecific antibody leads the activation of CD3 signaling pathway in Jurkat/NFAT-Luc cells by the B7-H3 on the A498 cells, promoting luciferase expression. Thereby, this assay provides a means to evaluate the efficacy of the bispecific antibodies in activating T cells through the interaction between B7-H3 and CD3 molecules.

In the study, A498 cells and Jurkat/NFAT-Luc cells were sequentially added to 96-well cell culture plate, and then the B7-H3×CD3 bispecific antibody was diluted to concentrations of 10 µg/mL, 2.5 µg/mL, 0.625 µg/mL, 0.15625 µg/mL, 0.0390 µg/mL, 0.0097 µg/mL, 0.0024 µg/mL, and 0.0006 µg/mL. The different concentrations of B7-H3×CD3 bispecific antibody were added to the culture plate, and incubated at 37 °C for 6 h. ONE-Glo Luciferase detection reagent was added, and chemiluminescence values were measured by using a multifunctional microplate reader. The logarithmic value of the antibody concentration was taken as the horizontal coordinate and the average chemiluminescence value was taken as the vertical ordinate for four-parameter fitting, plotting the dose-effect curves, obtaining the EC₅₀ value of each curve. The EC₅₀ represents the activity of B7-H3×CD3 bispecific antibody to activate T cells. 8H9×CD3 was used as a positive control antibody in the study.

The results are shown in FIG. 7. The data showed that the four strains of B7-H3×CD3 bispecific antibodies exhibited a superior activity on T cells activation, when co-incubated with B7-H3 positive tumor A498 cells and Jurkat/NFAT-Luc cells.

### (3) T-cell-mediated cytotoxicity of B7-H3×CD3 bispecific antibodies against B7-H3 positive tumor cells in vitro.

The CD3 binding arm of the B7-H3×CD3 bispecific antibody specifically binds to the CD3 complex on the surface of T cells, and the B7-H3 binding arm specifically binds to the B7-H3 molecule on the surface of tumor cells, resulting in the formation of an immune bridging link between the T cells and the tumor cells. This linkage activates the T cells and releases cytokines such as perforin and granzyme B, which eliminate the tumor cells. When tumor cell membrane is damaged, it results in increased cell membranes permeability. As a consequence, lactate dehydrogenase (LDH) in the cytoplasm is released into the culture supernatant. The lactate dehydrogenase catalyzed a dehydrogenation reaction to produce a red product formazan, which could produce an absorption peak at a wavelength of 490 nm. The lactate dehydrogenase released from the tumor cells can be quantified by measuring the absorbance value, and to calculate the T-cell-mediated cytotoxicity activity of the B7-H3×CD3 bispecific antibodies. The cytotoxicity killing was calculated as Cytotoxicity%=(sample OD value -SR)/(MR-SR)×100%, where SR= spontaneous release wells OD value (target cells + effector cells), and MR= maximal release wells OD value (target cells).

In the study, NCI-N87, A498, and HepG2 cells with different expression level of B7-H3 were used as the target cells, and the human PBMCs as the effector cells to detect the T-cell-mediated cytotoxicity activity of four strains B7-H3×CD3 bispecific antibody.

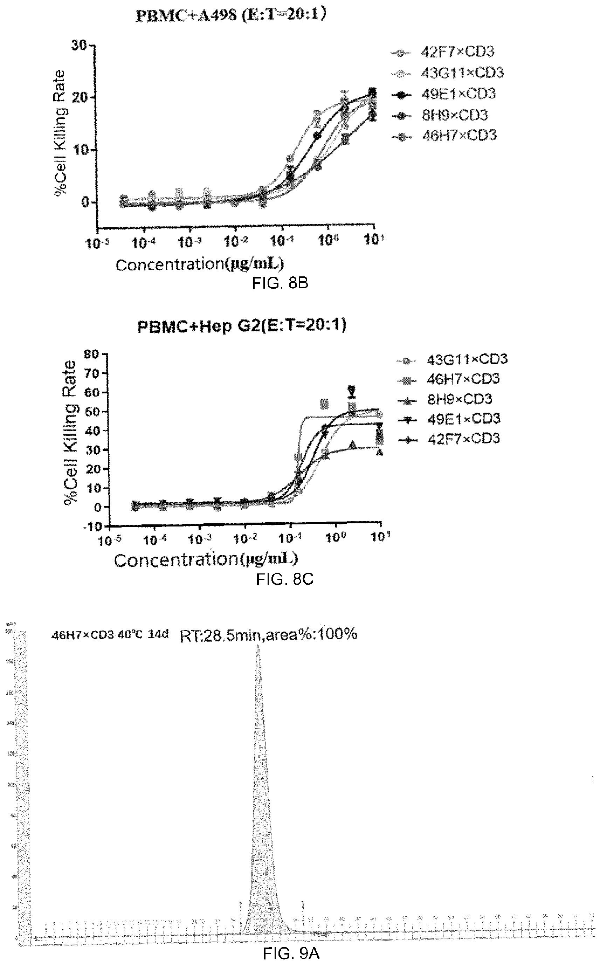

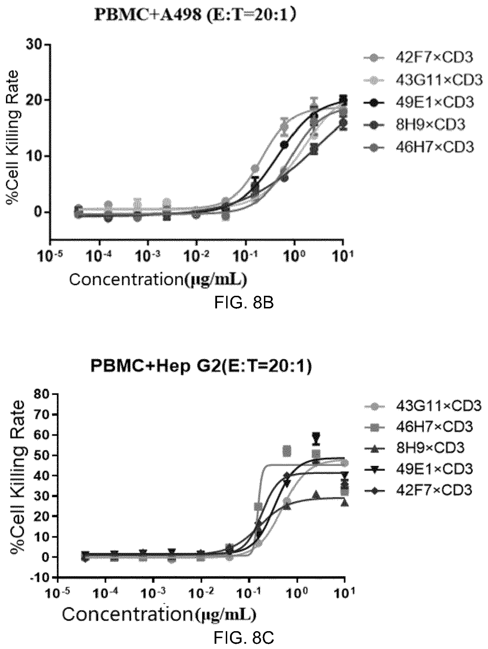

Freshly isolated human PBMCs were taken and the density was adjusted to 2.00×10⁶ cells/mL. NCI-N87, A498, and HepG2 cells were taken and the density was adjusted to 2.00×10⁵ cells/mL. B7-H3×CD3 bispecific antibody samples were diluted to 30 µg/mL with 1×PBS buffer solution (pH 7.4), then subjected to four-fold gradient dilution with a total of 10 gradients, wherein concentrations thereof successively were 30 µg/mL, 7.5 µg/mL, 1.875 µg/mL, 0.4688 µg/mL, 0.1172 µg/mL, 0.0293 µg/mL, 0.0073 µg/mL, 0.0018 µg/mL, 0.0005 µg/mL, and 0.0001 µg/mL. Sequentially 50 µL/well target cells, 100 µL/well PBMCs and 50 µL/well gradient dilution of the samples were added to the 96-well cell culture plate, mixed well so that the ratio of PBMCs to the target cells was 20:1. The starting concentration of the samples was 10 µg/mL. Control wells were replenished with 200 µL of phenol red-free RPMI 1640 culture medium. The plates were incubated in a 37 °C, 5% CO₂ incubator for 21±1h. Cytotoxicity was detected by the lactate dehydrogenase cytotoxicity kit (Beyotime). The logarithmic value of the antibody concentration was taken as the horizontal coordinate and the cytotoxicity value was taken as the vertical ordinate for four-parameter fitting, the dose-effect curves were plotted, obtaining the EC₅₀ value of each curve. The EC₅₀ represents the cytotoxicity activity of the B7-H3×CD3 bispecific antibodies against the target cells. Results are shown in FIG. 8A to FIG. 8C.

As shown in FIG. 8A to FIG. 8C, the B7-H3×CD3 bispecific antibodies constructed in the present study have relatively good T cell cytotoxicity against on the NCI-N87, A498, and HepG2 cells with different expression level of B7-H3.

### (4) Stability study of B7-H3×CD3 bispecific antibodies

To evaluate the stability of the B7-H3×CD3 bispecific antibodies at 40 °C for different periods of time, the sealed samples (1 mg/mL) were placed in a 40 °C incubator (BINDER KBF240). The purity of samples was analyzed by SEC-HPLC at corresponding time points (baseline (day 0), day 14). The parameter setting of SEC-HPLC were as follows:
Exclusion chromatography column: Superdex200 10/300 GL increase;
Mobile phase: 50 mM NaAc, 51 mM NaCl, 0.05 mM EDTA pH 5.5;
Flow rate: 0.5 mL/min;
UV detection wavelength: 280 nm;
Collection time: 35 min.

An AKTA pure 25 L1 chromatograph was utilized with UNICORN software to record and calculate the proportion of remaining monomers.

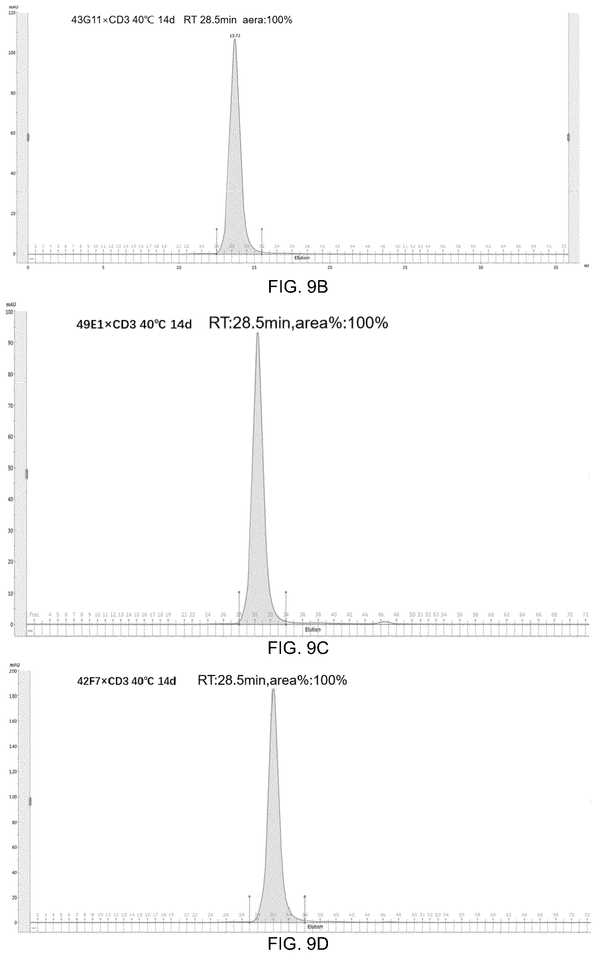

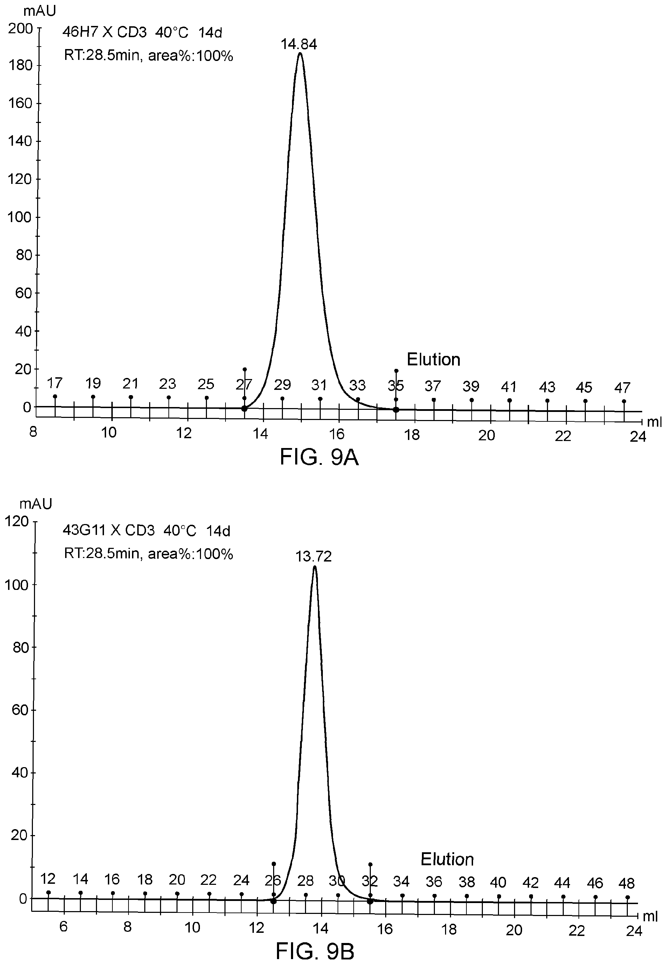

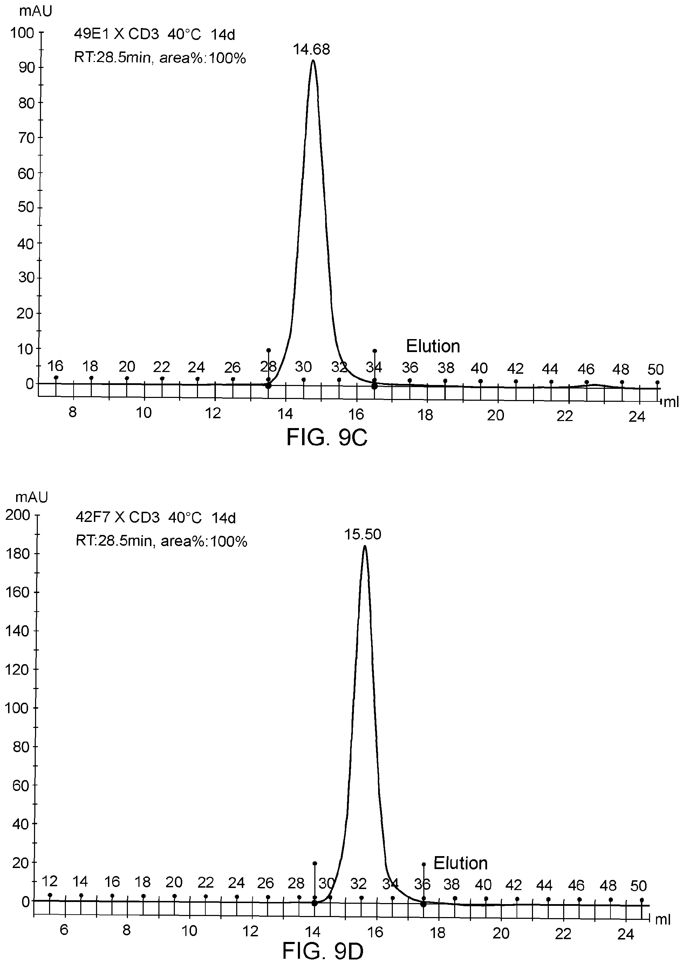

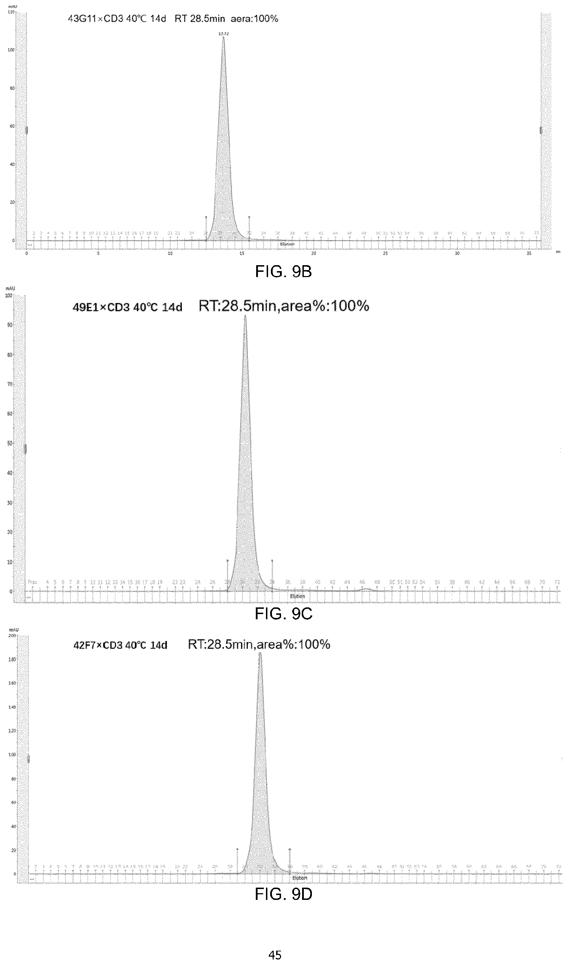

As shown in FIG. 9A to FIG. 9D, FIG. 9A shows the results of the purity of 46H7×CD3 bispecific antibody detected by SEC-HPLC after being placed at 40 °C for 14 days, in which retention time of the 46H7×CD3 bispecific antibody is 28.5 min, the peak area ratio is 100%, and its purity is greater than 98%. FIG. 9B shows results of the purity of 43G11×CD3 bispecific antibody detected by SEC-HPLC after being placed at 40 °C for 14 days, in which retention time of the 43G11×CD3 bispecific antibody is 28.5 min, the peak area ratio is 100%, and its purity is greater than 98%. FIG. 9C shows results of the purity of 49E1×CD3 bispecific antibody detected by SEC-HPLC after being placed at 40 °C for 14 days, in which retention time of the 49E1×CD3 bispecific antibody is 28.5 min, the peak area ratio is 100%, and its purity is greater than 98%. FIG. 9D shows results of the purity of 42F7×CD3 bispecific antibody detected by SEC-HPLC after being placed at 40 °C for 14 days, in which retention time of the 42F7×CD3 bispecific antibody is 28.5 min, the peak area ratio is 100%, and its purity is greater than 98%. The results demonstrated that, under the experimental condition of 40°C, the multimer ratio of the antibody did not increase over time. This suggests that the bispecific tetravalent antibody exhibits favorable thermal stability and is considered suitable for further development.

### (5) Anti-tumor activity of the B7-H3×CD3 bispecific antibody in mice model

According to the results of the foregoing examples, one of the B7-H3×CD3 bispecific antibodies (42F7×CD3) was selected to analyze the anti-tumor activity in xenograft model.

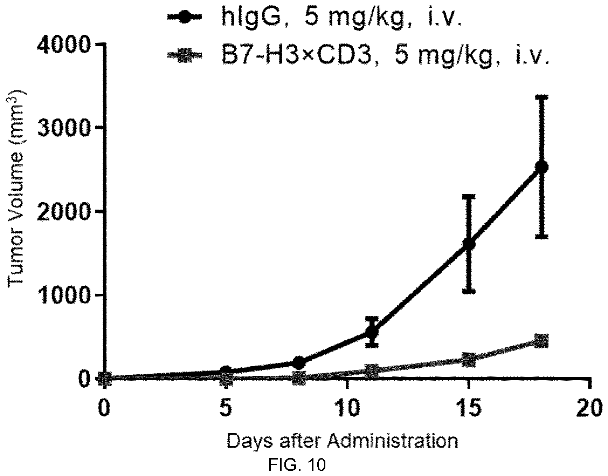

8-week-old female NOD-SCID mice (Beijing Vital River Laboratory Animal Technology Co., Ltd.) were selected, and MC38/hB7-H3 cells in a log growth phase and freshly isolated PBMCs were collected and inoculated subcutaneously into the right dorsal side of mice at 100 µL per mice. The mice were grouped on the next day of inoculation. The day of inoculation was defined as D0 day. Dosing was started on the D0 day. The study was divided into two groups, six mice in each group, respectively, the isotype control hlgG group (5 mg/kg, i.v.) and the B7-H3×CD3 group (5 mg/kg, i.v.). The mice were administered via tail intravenous injection (i.v.) on D1, D5, D8, D11, and D15. Length (mm) and width (mm) of tumors and body weights were monitored. After the dosing, a tumor volume was measured and calculated by: Tumor volume (mm³) =0.5×(Tumor Length ×Tumor Width²). The tumor proliferation rate (T/C%) and tumor inhibition rate (tumor growth inhibition, TGI) were calculated according to the tumor volume. The Anti-tumor activity was evaluated according to tumor growth inhibition (TGI), and the results are shown in FIG. 10.

As shown in FIG. 10, the B7-H3×CD3 has a significant activity of inhibiting tumor growth in mouse tumor models of MC38/hB7-H3 xenograft models, subcutaneously established in NOD/SCID mice with PBMC- reconstituted human immune system (the B7-H3×CD3 bispecific antibodies showed a strong anti-tumor activity on B7-H3 positive tumor cells in mice model).

In summary, the B7-H3 antibodies in the present disclosure can bind to the B7-H3 antigen with the high affinity, the affinity of the B7-H3 antibodies is further improved by mutating the heavy chain complementarity determining regions, and the B7-H3 antibodies can efficiently mediate the PBMC to kill the B7-H3 positive tumors. Meanwhile, by constructing the B7-H3×CD3 bispecific antibodies using the B7-H3 antibodies in the present disclosure, the bispecific antibodies have high affinity and stability, can effectively mediate the PBMC to kill the tumor cells, and have a significant effect in inhibiting the tumor growth.

The applicant states that detailed methods of the present disclosure are illustrated through the above examples in the present disclosure, but the present disclosure is not limited to the detailed methods above, that is, it does not mean that the present disclosure must be implemented relying upon the detailed methods above. Those skilled in the art should know that any improvements on the present disclosure, equivalent substitutions of raw materials and addition of auxiliary components to products of the present disclosure, choices of modes, and the like, fall within the scope of protection and the disclosed scope of the present disclosure.

### Industrial Applicability

This invention provides a B7-H3 antibody and use thereof. The B7-H3 antibody provided in this invention can bind to the B7-H3 antigen and cells expressing the B7-H3 antibody with a high affinity, can efficiently mediate the PBMC to kill the B7-H3 positive tumors. The B7-H3×CD3 bispecific antibody is constructed using the B7-H3 antibody, which bispecific antibody has high affinity and stability, can effectively mediate the PBMC to kill the tumor cells, and has a significant effect in inhibiting the tumor growth. Therefore, the B7-H3 antibody provided in this invention has excellent applicability.

## Claims

1. A B7-H3 antibody, wherein heavy chain complementarity determining regions of the B7-H3 antibody comprise amino acid sequences as set forth in SEQ ID Nos. 6-8; and
light chain complementarity determining regions of the B7-H3 antibody comprise amino acid sequences as set forth in SEQ ID Nos. 14-16.

2. The B7-H3 antibody according to claim 1, wherein heavy chain variable regions of the B7-H3 antibody comprise an amino acid sequence as set forth in SEQ ID No. 1; and
light chain variable regions of the B7-H3 antibody comprise an amino acid sequence as set forth in SEQ ID No. 9.

3. An affinity-matured B7-H3 antibody, wherein the affinity-matured B7-H3 antibody is derived from the B7-H3 antibody according to claim 1 or 2 through mutation selection.

4. The affinity-matured B7-H3 antibody according to claim 3, wherein a heavy chain complementarity determining region CDR1 of the affinity-matured B7-H3 antibody comprises an amino acid sequence as set forth in any one of SEQ ID No. 6 or SEQ ID Nos. 17-25, a heavy chain complementarity determining region CDR2 comprises an amino acid sequence as set forth in any one of SEQ ID No. 7 or SEQ ID Nos. 26-37, and a heavy chain complementarity determining region CDR3 comprises an amino acid sequence as set forth in any one of SEQ ID No. 8 or SEQ ID Nos. 38-48; and
light chain complementarity determining regions of the affinity-matured B7-H3 antibody comprise amino acid sequences as set forth in SEQ ID Nos. 14-16.

5. The affinity-matured B7-H3 antibody according to claim 3 or 4, wherein light chain variable regions of the affinity-matured B7-H3 antibody comprise an amino acid sequence as set forth in SEQ ID No. 9; and heavy chain framework regions of the affinity-matured B7-H3 antibody comprise amino acid sequences as set forth in SEQ ID Nos. 2-5.

6. A bispecific antibody, wherein the bispecific antibody comprises two heavy chains and two light chains;
the heavy chains comprise an anti-B7-H3 heavy chain and an anti-CD3 heavy chain; the anti-B7-H3 heavy chain comprises a heavy chain of the B7-H3 antibody according to claim 1 or 2 or a heavy chain of the affinity-matured B7-H3 antibody according to any one of claims 3-5;
the light chains comprise an anti-B7-H3 light chain and an anti-CD3 light chain; the anti-B7-H3 light chain comprises a light chain of the B7-H3 antibody according to claim 1 or 2 or a light chain of the affinity-matured B7-H3 antibody according to any one of claims 3-5;
the heavy chains bind to the light chains by disulfide bonds; and
the anti-B7-H3 heavy chain binds to the anti-CD3 heavy chain through disulfide bonds.

7. The bispecific antibody according to claim 6, wherein a variable region of the anti-CD3 heavy chain comprises an amino acid sequence as set forth in SEQ ID No. 49; and
a variable region of the anti-CD3 light chain comprises an amino acid sequence as set forth in SEQ ID No. 9.

8. The bispecific antibody according to claim 6 or 7, wherein the bispecific antibody comprises an immunoglobulin Fab domain binding to the B7-H3, an immunoglobulin Fab domain binding to the CD3, and a heterodimeric Fc region;
the immunoglobulin Fab domain binding to B7-H3 comprises a variable region VH and a constant region CH1 of the anti-B7-H3 heavy chain, and a variable region and constant region of the anti-B7-H3 light chain;
the immunoglobulin Fab domain binding to the CD3 comprises a variable region VH and a constant region CH1 of the anti-CD3 heavy chain, and a variable region and constant region of the anti-CD3 light chain; and
the heterodimeric Fc region comprises an Fc fragment linked to the anti-B7-H3 heavy chain and an Fc fragment linked to the anti-CD3 heavy chain.

9. The bispecific antibody according to claim 8, wherein the Fc fragment linked to the anti-B7-H3 heavy chain comprises a human Fc fragment or a humanized Fc fragment.

10. The bispecific antibody according to claim 8 or 9, wherein CH3 of the Fc fragment linked to the anti-B7-H3 heavy chain contains T394D, P395D, and P396D mutations.

11. The bispecific antibody according to any one of claims 8-10, wherein CH3 of the Fc fragment linked to the anti-CD3 heavy chain contains P395K, P396K, and V397K mutations.

12. The bispecific antibody according to any one of claims 8-11, wherein CH2 of the Fc fragment linked to the anti-B7-H3 heavy chain contains L234A, L235A, and P329G mutations.

13. A biomaterial, wherein the biomaterial is any one selected from the group consisting of B1) to B6):
B1) a nucleic acid molecule encoding the antibody of any one of (b1) to (b3), or a heavy chain and/or a light chain in an antigen-binding domain of the antibody of any one of (b1) to (b3): (b1) is the B7-H3 antibody according to claim 1 or 2, (b2) is the affinity-matured B7-H3 antibody according to any one of claims 3-5, and (b3) is the bispecific antibody according to any one of claims 6-12;
B2) an expression cassette containing the nucleic acid molecule in B1);
B3) a recombinant vector containing the nucleic acid molecule in B1), or a recombinant vector containing the expression cassette in B2);
B4) a recombinant microorganism containing the nucleic acid molecule in B1), a recombinant microorganism containing the expression cassette in B2), or a recombinant microorganism containing the recombinant vector in B3);
B5) a cell line containing the nucleic acid molecule in B1), a cell line containing the expression cassette in B2), or a cell line containing the recombinant vector in B3); and
B6) a nucleic acid molecule encoding the antibody of any one of (b1) to (b3) or a variable region of a heavy chain and/or a variable region of a light chain in the antigen-binding domain of the antibody of any one of (b1) to (b3).

14. A pharmaceutical composition, wherein the pharmaceutical composition comprises any one or a combination of at least two selected from the group consisting of the B7-H3 antibody according to claim 1 or 2, the affinity-matured B7-H3 antibody according to any one of claims 3-5, or the bispecific antibody according to any one of claims 6-12.

15. Use of the B7-H3 antibody according to claim 1 or 2, the affinity-matured B7-H3 antibody according to any one of claims 3-5, the bispecific antibody according to any one of claims 6-12, or the pharmaceutical composition according to claim 14 in preparation of a drug for resisting a tumor,
wherein the tumor comprises any one or a combination of at least two selected from the group consisting of neurological system tumor, colorectal cancer, liver cancer, head and neck cancer, lung cancer, melanoma, pancreatic cancer, gastric cancer, kidney cancer, bladder cancer, breast cancer, ovarian cancer and prostate cancer.

16. Use of the B7-H3 antibody according to claim 1 or 2, the affinity-matured B7-H3 antibody according to any one of claims 3-5, the bispecific antibody according to any one of claims 6-12, or the pharmaceutical composition according to claim 14 in treatment of a tumor; and
preferably, the tumor comprises any one or a combination of at least two selected from the group consisting of neurological system tumor, colorectal cancer, liver cancer, head and neck cancer, lung cancer, melanoma, pancreatic cancer, gastric cancer, kidney cancer, bladder cancer, breast cancer, ovarian cancer and prostate cancer.

17. A method for treating diseases associated with a tumor, comprising administering to a subject in need thereof the B7-H3 antibody according to claim 1 or 2, the affinity-matured B7-H3 antibody according to any one of claims 3-5, the bispecific antibody according to any one of claims 6-12, or the pharmaceutical composition according to claim 14; and
preferably, the tumor comprises any one or a combination of at least two selected from the group consisting of neurological system tumor, colorectal cancer, liver cancer, head and neck cancer, lung cancer, melanoma, pancreatic cancer, gastric cancer, kidney cancer, bladder cancer, breast cancer, ovarian cancer and prostate cancer.
